# EUROPEAN PATENT SPECIFICATION

(11) **EP 2 066 667 B1**
(45) Date of publication and mention of the grant of the patent: **31.08.2011**
(21) Application number: 07803228.1
(22) Date of filing: 05.09.2007
(51) Int. Cl.: C07D 471/04

(54) **PROCESS FOR THE PREPARATION OF PYRIDO[2,1-A]ISOQUINOLINE DERIVATIVES COMPRISING OPTICAL RESOLUTION OF AN ENAMINE**
VERFAHREN ZUR HERSTELLUNG VON PYRIDO[2,1-A]ISOCHINOLINDERIVATEN, DAS DIE RACEMATSPALTUNG EINES ENAMINS UMFASST
MÉTHODE DE PRÉPARATION DE DÉRIVÉS DE PYRIDO[2,1-A]ISOQUINOLINE COMPRENANT UNE RÉSOLUTION OPTIQUE D'UNE ÉNAMINE

(30) Priority: 15.09.2006 EP 06120722
(43) Date of publication of application: 10.06.2009
(73) Proprietor: F. Hoffmann-La Roche AG, 4070 Basel (CH)
(72) Inventor: ABRECHT, Stefan, 4202 Duggingen (CH); ADAM, Jean-Michel, 68128 Rosenau (FR); FETTES, Alec, 8001 Zuerich (CH); HILDBRAND, Stefan, 4313 Moehlin (CH)
(74) Representative: Klostermeyer-Rauber, Dörte
(86) International application number: PCT/EP2007/059264
(87) International publication number: WO 2008/031749

(56) References cited:
- WO-A-2005/000848
- BROSSI, A. ET AL: "Synthetic experiments in the emetine series. The absolute configuration of (-)-2-dehydroemetine" HELVETICA CHIMICA ACTA , 45(6), 2219-26 CODEN: HCACAV; ISSN: 0018-019X, 1962, XP002461881

## Description

The present invention relates to a process for the preparation of pyrido[2,1-a] isoquinoline derivatives of the formula 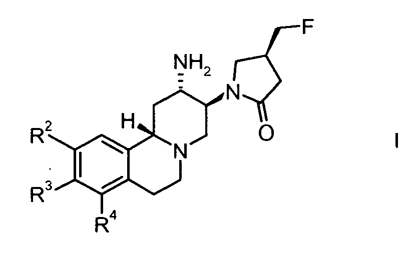 wherein
R², R³ and R⁴ are each independently selected from the group consisting of hydrogen, halogen, hydroxy, lower alkyl, lower alkoxy and lower alkenyl, wherein lower alkyl, lower alkoxy and lower alkenyl may optionally be substituted by a group consisting of lower alkoxycarbonyl, aryl and heterocyclyl, and the pharmaceutically acceptable salts thereof useful for the treatment and / or prophylaxis of diseases which are associated with DPP IV.

The pyrido [2,1-a] isoquinoline derivatives of the formula I are disclosed in PCT International Patent Appl. WO 2005/000848.

A major task in the synthesis of the compounds of formula I is the introduction of the chiral center in the pyrido [2,1-a] isoquinoline moiety, which in the current synthesis according to the PCT Int. Appl. WO 2005/000848 involves late stage racemate separation by chiral HPLC. Such a process is however difficult to manage on technical scale. The problem to be solved therefore was to find a suitable process alternative which allows to obtain the desired optical isomer in early stage of the process, which affords a higher yield and which can be conducted on technical scale.

It was found that with the process of the present invention, as outlined below, the problem could be solved.

Unless otherwise indicated, the following definitions are set forth to illustrate and define the meaning and scope of the various terms used to describe the invention herein.

The term "halogen" refers to fluorine, chlorine, bromine and iodine, with fluorine, bromine and chlorine being preferred.

The term "alkyl", alone or in combination with other groups, refers to a branched or straight-chain monovalent saturated aliphatic hydrocarbon radical of one to twenty carbon atoms, preferably one to sixteen carbon atoms, more preferably one to ten carbon atoms.

In this specification the term "lower" is used to mean a group consisting of one to six, preferably of one to four carbon atom(s).

Thus, the term "lower alkyl", alone or in combination with other groups, refers to a branched or straight-chain monovalent alkyl radical of one to six carbon atoms, preferably one to four carbon atoms. This term is further exemplified by radicals such as methyl, ethyl, n-propyl, isopropyl, n-butyl, s-butyl, isobutyl, t-butyl, n-pentyl, 3-methylbutyl, n-hexyl, 2-ethylbutyl and the like. Preferable lower alkyl residues are methyl and ethyl, with methyl being especially preferred.

The term "alkenyl" as used herein denotes an unsubstituted or substituted hydrocarbon chain radical having from two to six carbon atoms, preferably from two to four carbon atoms, and having one or two olefinic double bonds, preferably one olefinic double bond. Examples are vinyl, 1-propenyl, 2-propenyl (allyl) or 2-butenyl (crotyl).

The term "alkoxy" refers to the group R'-O-, wherein R' is alkyl. The term "lower-alkoxy" refers to the group R'-O-, wherein R' is a lower alkyl group as defined above. Examples of lower alkoxy groups are e.g. methoxy, ethoxy, propoxy, isopropoxy, butoxy, isobutoxy and hexyloxy, with methoxy being especially preferred.

The term "lower alkoxycarbonyl" refers to the group R'-O-C (O)-, wherein R' is a lower alkyl group as defined above.

The term "aryl" refers to an aromatic monovalent mono- or polycarbocyclic radical, such as phenyl or naphthyl, preferably phenyl, which may optionally be mono-, di- or tri-substituted, independently, by lower alkyl, lower alkoxy, halogen, cyano, azido, amino, di-lower alkyl amino or hydroxy.

The term "heterocyclyl" refers to a 5- or 6-membered aromatic or saturated N-heterocyclic residue, which may optionally contain a further nitrogen or oxygen atom, such as imidazolyl, pyrazolyl, thiazolyl, pyridyl, pyrimidyl, morpholino, piperazino, piperidino or pyrrolidino, preferably pyridyl, thiazolyl or morpholino. Such heterocyclic rings may optionally be mono-, di- or tri-substituted, independently, by lower alkyl, lower alkoxy, halogen, cyano, azido, amino, di-lower alkyl amino or hydroxy. Preferable substituent is lower alkyl, with methyl being preferred.

The term "pharmaceutically acceptable salts" embraces salts of the compounds of formula I with inorganic or organic acids such as hydrochloric acid, hydrobromic acid, nitric acid, sulphuric acid, phosphoric acid, citric acid, formic acid, maleic acid, acetic acid, fumaric acid, succinic acid, tartaric acid, methanesulphonic acid, salicylic acid, p-toluenesulphonic acid and the like, which are non toxic to living organisms. Preferred salts with acids are formates, maleates, citrates, hydrochlorides, hydrobromides and methanesulfonic acid salts; with hydrochlorides being especially preferred.

In detail, the process for the preparation of pyrido[2,1-a]isoquinoline derivatives of the formula 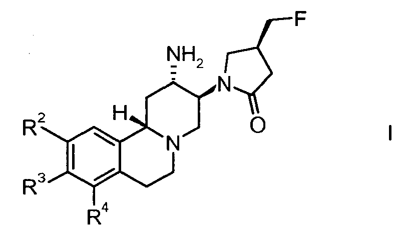 wherein R²; R³ and R⁴ are each independently selected from the group consisting of hydrogen, halogen, hydroxy, lower alkyl, lower alkoxy and lower alkenyl, wherein lower alkyl, lower alkoxy and lower alkenyl may optionally be substituted by a group selected from lower alkoxycarbonyl, aryl and heterocyclyl,
is comprising one or more of the steps a), b), c) or d) wherein step a) comprises the optical resolution of an enamine of the formula 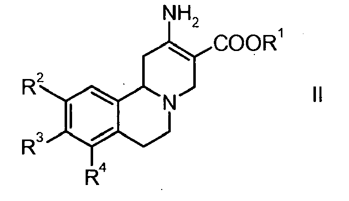 wherein R², R³ and R⁴ are as defined above and R¹ is lower alkyl or benzyl, in the presence of an optical active resolving agent to form the (S)-enamine salt of the formula 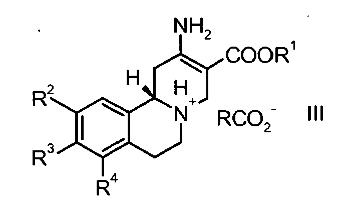 wherein R¹, R², R³ and R⁴ are as defined above and RCO₂⁻ is the conjugate base of the resolving agent;
step b) comprises the transformation of the (S)-enamine salt of formula III into the ester of formula 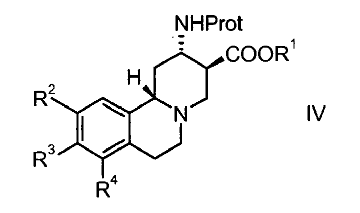 wherein R¹, R², R³ and R⁴ are as above and Prot stands for an amino protecting group;
step c) comprises amidation of the ester of formula IV to form the amide of formula 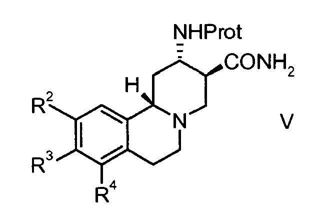 wherein R², R³, R⁴ and Prot are as defined above and
step d) comprises degradation of the amide of formula V to form the amine of formula 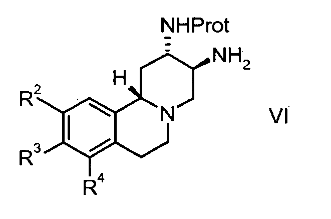 wherein R², R³, R⁴ and Prot are as defined above; wherein

the process of the present invention comprises step a) as defined before and wherein the optical resolution in step a) is a crystallization-induced dynamic resolution.

In another embodiment, the process of the present invention comprises step a) followed by step b) as defined before.

In yet another embodiment of the present invention, the process comprises steps a) to d) together.

In a further embodiment, the present invention relates to the process for the preparation of pyrido[2,1-a]isoquinoline derivatives of the formula I, wherein the steps b) and c) are carried out without isolation of the intermediate IV.

Preferably R¹ is lower alkyl. More preferably, R¹ is methyl, ethyl or isopropyl. Most preferably, R¹ is ethyl.

Preferably R², R³ and R⁴ are independently selected from hydrogen, lower alkyl and lower alkoxy. Especially preferred are those compounds, wherein R² and R³ are lower alkoxy and R⁴ is hydrogen.

Step a) comprises the optical resolution of an enamine of the formula 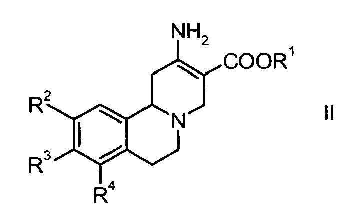 wherein R², R³ and R⁴ are as defined above and R¹ is lower alkyl or benzyl, in the presence of an optically active resolving agent to form the (S)-enamine salt of the formula 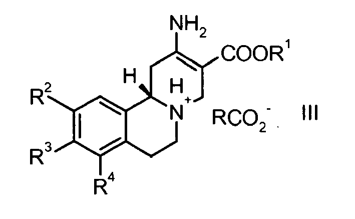 wherein R¹, R², R³ and R⁴ are as defined above and RCO₂⁻ is the conjugate base of the resolving agent.

The enamine of formula II can be synthesized from commercially available precursors according to scheme 1 below.

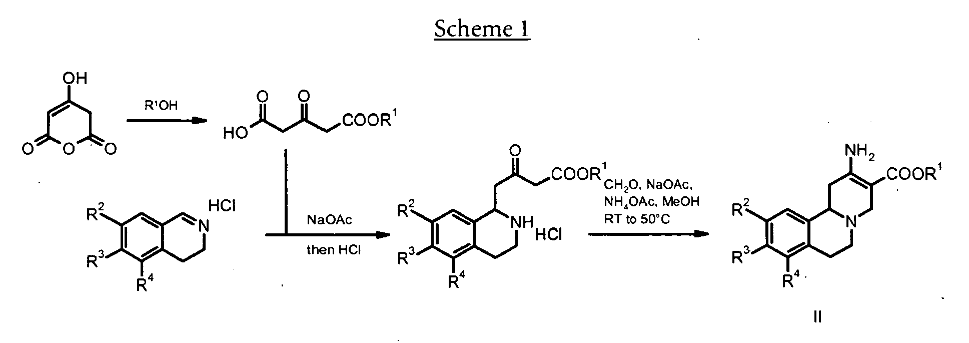

Suitable resolving agents of the formula R-CO₂H are tartaric acid derivatives of the formula 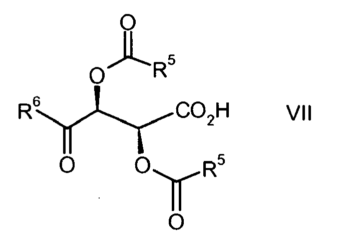 wherein
- R⁵: is selected from the group consisting of unsubstituted phenyl, phenyl substituted by one, two, or three groups independently selected from lower alkyl, lower alkoxy and halogen, lower alkyl, benzyl, wherein the phenyl ring is unsubstituted or substituted by one, two, or three groups independently selected from lower alkyl, lower alkoxy and halogen, and -NH-phenyl, wherein the phenyl ring is unsubstituted or substituted by one, two, or three groups independently selected from lower alkyl, lower alkoxy and halogen; and
- R⁶: is selected from the group consisting of hydroxy, lower alkoxy and -NR⁷R⁸, wherein R⁷ and R⁸ independently from each other are lower alkyl.

Preferably, R⁵ is selected from the group consisting of unsubstituted phenyl, phenyl substituted by one, two, or three groups independently selected from lower alkyl, lower alkoxy and halogen, and -NH-phenyl, wherein the phenyl ring is unsubstituted or substituted by one, two or three groups independently selected from lower alkyl, lower alkoxy and halogen.

More preferably, R⁵ is unsubstituted phenyl or phenyl substituted by one, two, or three groups independently selected from lower alkyl, lower alkoxy and halogen.

Preferably, R⁶ is hydroxy or -N(CH₃)₂. More preferably, R⁶ is hydroxy.

Examples of preferred compounds of formula VII are selected from the group consisting of (+)-O,O'-dibenzoyl-D-tartaric acid, (+)-O,O'-dibenzoyl-D-tartaric acid mono dimethylamide, (+)-O,O'-Di-p-toluoyl-D-tartaric acid and [S-(R*,R*)]-2,3-bis[[(phenylamino)carbonyl]oxy]-butanedioic acid,

Preferred resolving agent is (+)-O,O'-dibenzoyl-D-tartaric acid.

The term "conjugate base of the resolving agent" means the corresponding anions selected from above acids of formula VII, thus anions having the formula VIII 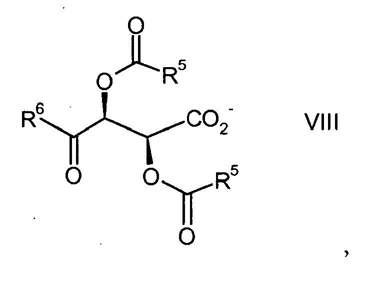 wherein R⁵ and R⁶ are as defined above.

The optical resolution according to step a) follows the principle of a crystallization-induced dynamic optical resolution (CIDR). In the classical optical resolution the desired isomer crystallizes and the undesired isomer remains in the solution. The yield of desired isomer can achieve a maximum of 50 % only. The concept of dynamic resolution is based on the continuous racemisation of the solved undesired isomer and the continuous crystallisation of the desired isomer. The yield of the desired isomer can thus reach a maximum of 100%.

The optical resolution or the crystallization-induced dynamic optical resolution is usually performed in a solvent selected from water, methanol, ethanol, isopropanol, acetone, tetrahydrofuran, ethyl acetate, toluene or mixtures thereof. The selection depends on the resolving agent.

For the dynamic optical resolution at least 1 equivalent of the resolving agent is required for complete conversion.

The process temperature is as a rule kept in a range of 40 °C to reflux temperature, preferably in a range of 55 °C to 65 °C.

The crystals of the (S)-enamine salt can be separated from the reaction mixture by filtration and drying.

In a preferred embodiment the optical resolution in step a) is performed with the enamine of formula II wherein R¹ is methyl, ethyl, isopropyl or benzyl, preferably R¹ is ethyl.

The (S)-enamine salts of formula III are novel compounds and accordingly are a further embodiment of the present invention.

Preferred (S)-enamine salts of formula III are:
(S)-2-amino-9,10-dimethoxy-1,6,7,11b-tetrahydro-4*H*-pyrido[2,1-a]isoquinoline-3-carboxylic acid ethylester, (2*S*,3*S*)-bis-benzoyloxy-succinic acid salt,
(*S*)-2-amino-9,10-dimethoxy-1,6,7,11b-tetrahydro-4*H*-pyrido[2,1-a]isoquinoline-3-carboxylic acid methyl ester, (2*S*,3*S*)-bisbenzoyloxy-succinic acid salt,
(*S*)-2-amino-9,10-dimethoxy-1,6,7,11b-tetrahydro-4*H*-pyrido[2,1-a]isoquinoline-3-carboxylic acid isopropyl ester, (2*S*,3*S*)-bisbenzoyloxy-succinic acid salt, and (*S*)-2-amino-9, 10-dimethoxy-1,6,7,11b-tetrahydro-4*H*-pyrido[2,1-a]isoquinoline-3-carboxylic acid ethyl ester, (2*S*,3*S*)-bis-benzyloxy-*N,N*-dimethyl-succinamic acid salt.

Step b) comprises the transformation of the (S)-enamine salt of formula III by a hydride reduction under acidic conditions followed by the introduction of an amino protecting group to the ester of formula 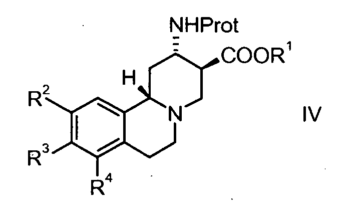 wherein R¹, R², R³ and R⁴ are as above and Prot stands for an amino protecting group;

This transformation produces the stereoisomer of formula IV with high selectivity.

The hydride reduction is performed with a reducing agent selected from sodium borohydride, lithium borohydride and sodium cyanoborohydride, preferably the reducing agent is sodium borohydride.

As a rule the (S)-enamine salt of formula III, suspended in a suitable acid, such as trifluoroacetic acid, mono-, di- or trichloroacetic acid, or acetic acid and an organic solvent, such as tetrahydrofuran (THF) or methyltetrahydrofuran (MeTHF), is added to a mixture of the reducing agent and a suitable solvent.

The (S)-enamine can also be added to a mixture of sodium borohydride and trifluoro acetic acid in the organic solvent.

The reaction temperature is as a rule kept in a range of -40 °C to 30 °C, preferably in a range of -20 °C to 25 °C.

The free amine can be separated by a work-up procedure known to the skilled in the art, for instance by extraction of the basified reaction mixture with a suitable organic solvent, common washing procedures and finally by removing the solvent.

Introduction of the amino protecting group can be effected following procedures well known to the skilled in the art.

The term "amino protecting group" or "Prot" refers to any substituents conventionally used to hinder the reactivity of the amino group. Suitable amino protecting groups and its introduction are described in Green T., "Protective Groups in Organic Synthesis", Chapter 7, John Wiley and Sons, Inc., 1991, 309-385. Suitable amino protecting groups are trichloroethoxycarbonyl, benzyloxycarbonyl (Cbz), chloroacetyl, trifluoroacetyl, phenylacetyl, formyl, acetyl, benzoyl, tert-butoxycarbonyl (BOC), para-methoxybenzyloxycarbonyl, diphenylmethoxycarbonyl, phthaloyl, succinyl, benzyl, diphenylmethyl, triphenylmethyl (trityl), methanesulfonyl, para-toluenesulfonyl, pivaloyl, trimethylsilyl, triethylsilyl, triphenylsilyl, and the like, whereby tert-butoxycarbonyl (Boc) is preferred.

In a preferred embodiment step b) comprises the manufacture of ester IV wherein R² and R³ are methoxy, R⁴ is hydrogen and R¹ and Prot are as defined above.

More preferably, step b) comprises the manufacture of ester IV wherein R¹ is ethyl, R² and R³ are methoxy, R⁴ is hydrogen and Prot is Boc.

The esters of formula IV are novel compounds and accordingly are a further embodiment of the present invention.

Preferred esters of formula IV are:
(2S,3S,11bS)- 2-tert-Butoxycarbonylamino-9,10-dimethoxy-1,3,4,6,7,11b-hexahydro-2H-pyrido[2,1-a]isoquinoline]-3-carboxylic acid ethyl ester, and
(2S,3S,11bS)-2-tert-Butoxycarbonylamino-9,10-dimethoxy-1,3,4,6,7,11b-hexahydro-2H-pyrido[2,1-a]isoquinoline]-3-carboxylic acid methyl ester.

Step c) comprises amidation of the ester of formula IV to form the amide of formula 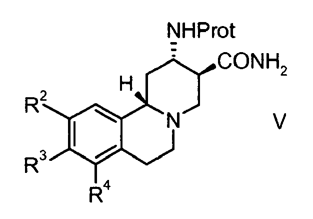 wherein R², R³, R⁴ and Prot are as defined above.

The amidation is usually performed with as suitable amidating agent, such as formamide/ sodium methoxide (NaOMe), formamide/ sodium ethoxide (NaOEt), acetamide/ sodium methoxide or acetamide/ sodium ethoxide.

The reaction can be effected in an organic solvent, such as THF, MeTHF, methanol, DMF, dioxane at temperatures of 10 °C to 70 °C, preferably of 20 °C to 45 °C.

In a preferred embodiment step c) comprises the manufacture of amide V wherein R² and R³ are methoxy, R⁴ is hydrogen and Prot is an amino protecting group as defined above.

In a preferred embodiment step c) comprises the manufacture of amide V wherein R² and R³ are methoxy, R⁴ is hydrogen and Prot is Boc.

Step d) comprises degradation of the amide of formula V to form the amine of formula 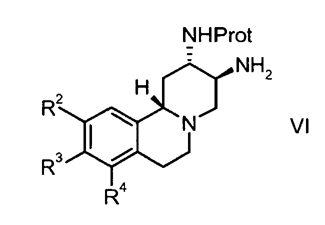 wherein R², R³, R⁴ and Prot are as above.

The degradation of the amide of formula V in step d) is performed according to the principles of the Hofmann-degradation using oxidizing agents selected from PIDA (iodosobenzene diacetate), PIFA (iodosobenzene bistrifluoracetate) or iodosobenzene bistrichloroacetate. Preferred oxidizing agents are PIDA (iodosobenzene diacetate) and PIFA (iodosobenzene bistrifluoracetate).

Usually the reaction is performed in a suitable solvent such as THF, acetonitrile, water or mixtures thereof and in the presence of excess of base such as for example sodium hydroxide or potassium hydroxide and at a reaction temperature in the range of 0 °C to 70 °C, preferably at 10 °C to 30 °C.

Work up and isolation of the amide of formula V can be carried out according to methods known to the skilled in the art.

In a preferred embodiment, step d) comprises the manufacture of amine VI wherein R² and R³ are methoxy, R⁴ is hydrogen and Prot is an amino protecting group as defined above.

More preferably, step d) comprises the manufacture of amine VI wherein R² and R³ are methoxy, R⁴ is hydrogen and Prot is Boc.

In a further embodiment, the invention relates to the preparation of amines of the formula VI. These compounds are useful intermediates for the preparation of DPP-IV inhibitors as disclosed in PCT International Patent Appl. WO 2005/000848. More preferably, the invention relates to a process for the preparation of (2S,3S,11bS)-(3-amino-9,10-dimethoxy-1,3,4,6,7,11b-hexahydro-2H-pyrido[2,1-a]isoquinolin-2-yl)]-carbamic acid tert-butyl ester.

### Further steps:

According to still another embodiment (Scheme 2, below) the (S)-4-fluoromethyl-dihydro-furan-2-one (VII) is directly coupled with the amino-pyrido [2,1-a] isoquinoline derivative (VI) to form the hydroxymethyl derivative of the pyrido [2,1-a] isoquinoline (VIII), which is then subsequently cyclized to the fluoromethyl-pyrrolidin-2-one derivative (IX). The latter can be deprotected to yield the desired pyrido [2,1-a] isoquinoline derivative (I).

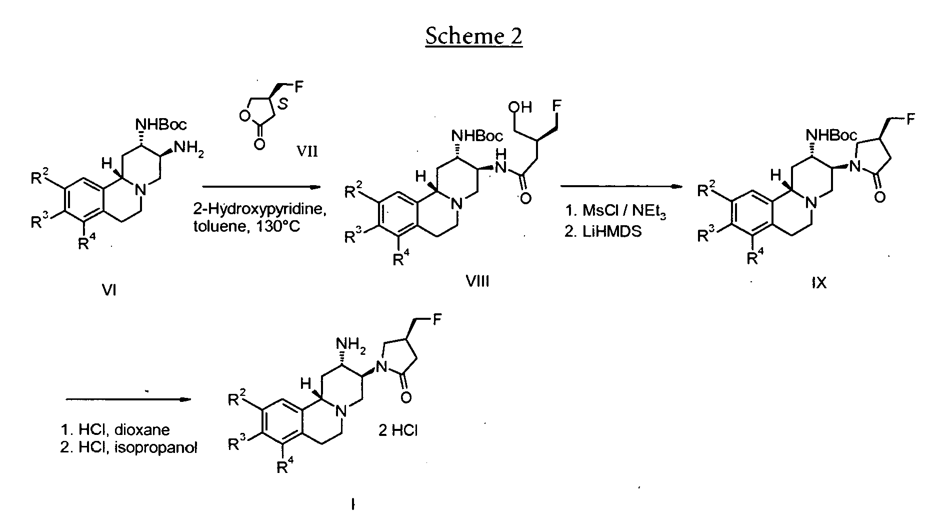

In a further preferable embodiment, the process for the preparation of (S)-1-((2S,3S,11bS)-2-amino-9,10-dimethoacy-1,3,4,6,7,11b-hexahydro-2H-pyrido[2,1-a]isoquinolin-3-yl)-4-fluoromethyl-pyrrolidin-2-one or of a pharmaceutically acceptable salt thereof comprises the subsequent steps:
e) coupling of the (2*S*,3*S*,11*bS*)-3-amino-9,10-dimethoxy-1,3,4,6,7,11b-hexahydro-2H-pyrido[2,1-a]isoquinolin-2-yl)-carbamic acid tert-butyl ester (amine of formula VI, wherein R² and R³ are methoxy, R⁴ is hydrogen and Prot is Boc) with the (S)-4-fluoromethyl-dihydro-furan-2-one of formula 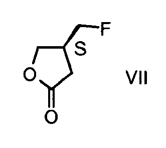
f) cyclization of the obtained (2*S*,3*S*,11*bS*)-3-(3-fluoromethyl-4-hydroxy-butyrylamino)-9,10-dimethoxy-1,3,4,6,7,11b-hexahydro-2H-pyrido[2,1-a]isoquinolin-2-yl]-carbamic acid tert-butyl ester in the presence of a base, and
g) deprotecting the obtained (2*S*,3*S*,11*bS*)-3-((4*S*)-fluoromethyl-2-oxo-pyrrolidin-1-yl)-9,10-dimethoxy-1,3,4,6,7,11b-hexahydro-2H-pyrido[2,1-a]isoquinolin-2-yl] -carbamic acid tert-butyl ester.

The pyrido [2,1-a] isoquinoline derivatives of formula (II) as disclosed in the PCT Int. Application WO 2005/000848 are useful for the treatment and/or prophylaxis of treatment and / or prophylaxis of diseases which are associated with DPP IV such as diabetes, particularly non-insulin dependent diabetes mellitus, and/or impaired glucose tolerance, as well as other conditions wherein the amplification of action of a peptide normally inactivated by DPP-IV gives a therapeutic benefit. Surprisingly, the compounds of the present invention can also be used in the treatment and/or prophylaxis of obesity, inflammatory bowel disease, Colitis Ulcerosa, Morbus Crohn, and/or metabolic syndrome or β-cell protection. Furthermore, the compounds of the present invention can be used as diuretic agents and for the treatment and/or prophylaxis of hypertension. Unexpectedly, the compounds of the present invention exhibit improved therapeutic and pharmacological properties compared to other DPP-IV inhibitors known in the art, such as e.g. in context with pharmacokinetics and bioavailability.

The following examples shall illustrate the invention without limiting it. The examples which do not fall under the scope of the claims, represent reference examples.

### Examples

### Abbreviations

| | |
|---|---|
| DMF | N,N-Dimethylformamide |
| MeOH | Methanol |
| EtOH | Ethanol |
| TBME | tert.-Butylmethylether |
| THF | Tetrahydrofuran |
| MeTHF | Methyltetrahydrofuran |
| RT | Room Temperature |
| ((R)-3,5-tBu-MeOBIPHEP) | (6,6'-Dimethoxy[1,1'-biphenyl]-2,2'-diyl)bis(bis(3,5-di-*tert.*-butylphenyl)phosphine |

### Synthesis of Precursor Compounds

### A1) Synthesis of (±)-1-(3-isopropoxycarbonyl-2-oxo-propyl)-6,7-dimethoxy-1,2,3,4-tetrahydro-isoquinolinium chloride (3c)

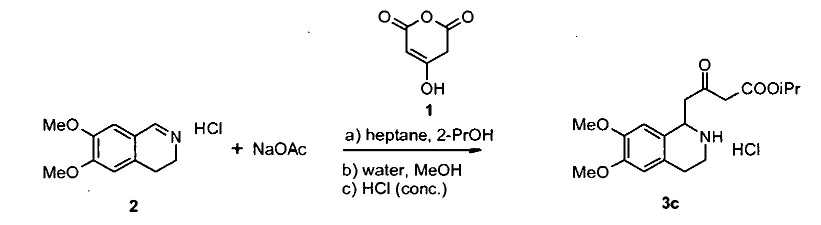

In a 100-ml two-necked round bottom flask equipped with a mechanical stirrer, an addition funnel and an argon in/outlet, 5.0 g of 1 was suspended in 19 ml of heptane at room temperature. 19 ml of 2-PrOH was added over 15 minutes and stirring was continued for 1 h. A 200-ml four-necked round bottom flask equipped with a mechanical stirrer, an addition funnel and an argon in/outlet, was charged with 7.4 g of 2, 268.0 mg of NaOAc and 1.9 ml of H₂O in 56 ml of 2-PrOH. To this mixture was added over 1.5 h the previously prepared emulsion and, after 1 h, 333 µL of conc. aqueous HCl was added. 55 ml of heptane was added over 30 minutes. The yellow suspension was stirred for 2 h at room temperature, filtered and washed portionwise with 12 ml of 2-PrOH and 24 ml of heptane (cooled to 0 °C). Evaporation of the solvent and drying under high vacuum gave 10.23 g (84%) of 3c as an off-white solid.

The cyclic anhydride of formula 1 used as reagent was prepared as follows:
2.13L acetic anhydride and 3L acetic acid were charged at room temperature in the reaction vessel. The solution was cooled to 8 °C to 10 °C and 2 kg of 1,3-acetone dicarboxylic acid were added. The reaction mixture was stirred 3h at 8 °C to 10 °C. After a reaction time of about 1.5h, a solution was almost obtained, upon which crystallization of the product started. After a reaction time of 3h at 8 to 10°C, the suspension was filtered. The crystalls were washed with 4L toluene and dried at 45 °C / 10 mbar to 20 mbar until constant weight to yield 1.33 kg of cyclic anhydride **1** (80% yield).

### A2) Synthesis of (±)-1-(3-ethoxycarbonyl-2-oxo-propyl)-6,7-dimethoxy-1,2,3,4-tetrahydro-isoquinolinium chloride (3a)

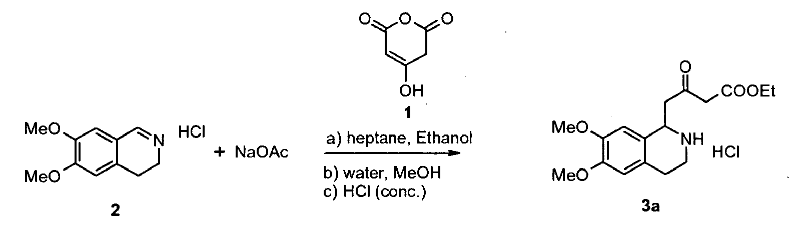

250 g of cyclic anhydride 1 was charged in the reaction vessel followed by 925 mL of heptane. 925 mL Ethanol were added over 15min to the suspension, keeping the temperature between 20-25 °C. After 1h reaction, the resulting solution was added over 1.5h to a solution consisting of 370 g of imine hydrochloride 2, 13.33 g sodium acetate, 2.77L ethanol and 93mL water, keeping the temperature between 20-25 °C. The product started to crystallize during the course of the reaction. After 1.5 h reaction, 16.48 mL of 37% HCl_{aq} were added followed by the addition of 2.75 L of heptane over 30min. The yellow suspension was stirred 2h at room temperature and filtered. The filter cake was washed with a cold (0°C) mixture of 599mL ethanol and 1.2 L of heptane. The crystals were dried at 50 °C under 10 mbar until constant weight to yield 534 g of amine hydrochloride 3a (88% yield, corrected for HPLC purity and residual solvent content).

### A3) Synthesis of (±)-1-(3-methoxycarbonyl-2-oxo-propyl)-6,7-dimethoxy-1,2,3,4-tetrahydro-isoquinolinium chloride

In a 100-ml two-necked round bottom flask equipped with a mechanical stirrer, an addition funnel and an argon in/outlet, 5.0 g of 1was suspended in 19 ml of heptane at room temperature. 19 ml of MeOH was added over 15 minutes and stirring was continued for 1 h. A 200-ml four-necked round bottom flask equipped with a mechanical stirrer, an addition funnel and an argon in/outlet was charged with 7.4 g of 2, 268.0 mg of NaOAc and 1.9 ml of H₂O in 56 ml of MeOH. To this mixture was added over 1.5 h the previously prepared emulsion and, after 1 h, 333 µl of conc. aqueous HCl was added. 55 ml of heptane was added over 30 minutes. The yellow suspension was stirred for 2 h at room temperature, filtered and washed portionwise with 12 ml of MeOH and 24 ml of heptane (cooled to 0 °C). Evaporation of the solvent and drying under high vacuum gave 9.37 g (84%) of 3b as an off-white solid.

### B1) Synthesis of (±)-2-amino-9,10-dimethoxy-1,6,7,11b-tetrahydro-4H-pyrido[2,1-a] isoquinoline-3-carboxylic acid methyl ester (4b)

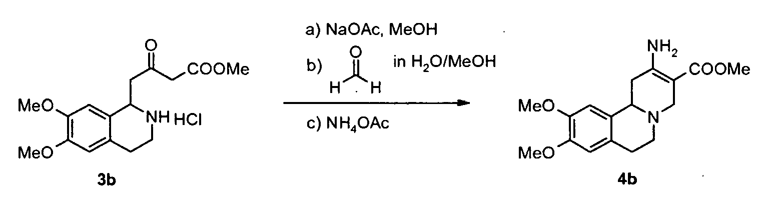

In a 100-ml two-necked round-bottom flask equipped with a magnetic stirrer, an addition funnel and an argon in/outlet, 6.0 g of 3b was suspended in 90 ml MeOH at room temperature and 1.44 g of NaOAc was added. The suspension was warmed till everything was dissolved, then added over 30 min at room temperature to a 200-ml four-necked round bottom flask equipped with a mechanical stirrer, a thermometer, an addition funnel, a reflux condenser and an argon in/outlet, containing 30 ml of MeOH and 1.4 ml of formaldehyde solution (13.3 M in MeOH/H₂O). 3 h later, 4.04 g of NH₄OAc was added then the solution was warmed to 47 °C. After 3 h the volatiles were removed and the residue was dissolved in 50 ml of CH₂Cl₂ and 25 ml of H₂O followed by slow addition of 40 ml of sat. aq. NaHCO₃. The organic phase was separated and washed twice with brine. The combined aqueous phase was extracted four times with 20 ml of CH₂Cl₂, the organic phases were collected and dried over MgSO₄. Filtration and evaporation of the solvent gave 5.6g of crude **4b** as red foam.

### B2) Synthesis of (±)-2-amino-9,10-dimethoxy-1,6,7,11b-tetrahydro-4H-pyrido[2,1-a]isoquinoline-3-carboxylic acid isopropyl ester (4c)

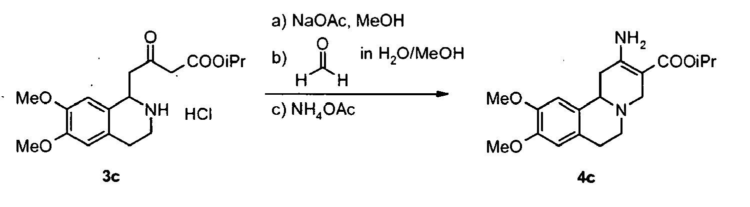

In a 100-ml two-necked round bottom flask equipped with a magnetic stirrer, an addition funnel and an argon in/outlet, 6.0 g of 3c was suspended in 90 ml of MeOH at room temperature and 1.44 g of NaOAc was added. The resulting mixture was transferred into an addition funnel and added over 0.5 h at room temperature to a 200-ml four-necked round-bottom flask equipped with a mechanical stirrer, a thermometer, a reflux condenser and an argon in/outlet, containing 30 ml of MeOH and 1.3 ml of formaldehyde solution (13.3 M in MeOH/H₂O). 3 h later, 3.73 g of NH₄OAc was added and the solution was warmed to 47 °C. After 3 h the volatiles were removed and the residue was dissolved in 50 ml of CH₂Cl₂ and 25 ml of H₂O, followed by slow addition of 38 ml of sat. aq. NaHCO₃. The organic phase was separated and washed twice with brine. The combined aqueous phases were extracted four times with 20 ml of CH₂Cl₂. The combined organic phases were dried over MgSO₄. Removal of the solvent and drying on high vacuum gave 4.98 g of 4c as red foam, which was used without further purification.

### B3) Synthesis of (±)-2-amino-9,10-dimethoxy-1,6,7,11b-tetrahydro-4H-pyrido[2,1-a]isoquinoline-3-carboxylic acid ethyl ester (4a)

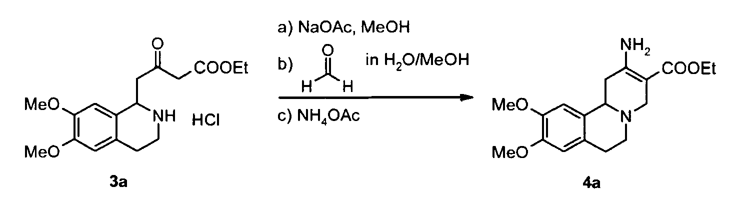

480 g of amine hydrochloride 3a were charged in the reaction vessel followed by 7.2 L methanol and 108.9 g sodium acetate. The obtained solution was added over 25 min, keeping the temperature between 20-22 °C, to a solution of 106.6 mL 36% aqueous formaldehyde in 2.4 L methanol. After 2.5h reaction, 306.9 g ammonium acetate were added and the reaction mixture was heated to 45-50 °C. After stirring overnight, the solution was concentrated to a thick oil. 4.0 L dichloromethane were added followed by 2.0 L water. 3.0 L 10% aqueous NaHCO₃ were slowly added. The organic phase was separated and washed with 3.0 L 10% aqueous NaCl. The aqueous phases were re-extracted sequentially with 3.6 L dichloromethane. The combined organic phases were concentrated and re-dissolved at reflux in 1.32 L methanol. The solution was cooled to 0°C over 8h, stirred 8h at 0 °C and 5h at -25 °C, after which the suspension was filtered. The filter cake was washed in portions with in total 800 mL cold (-25°C) methanol and 300 mL cold (-25°C) heptane. The crystals were dried at 45°C under 3 mbar to give 365 g enamino ester **4a** (73% yield, corrected for HPLC purity and residual solvent).

### Example 1a

Procedure for the optical resolution of (±)-enamine 1 using (+)-O,O'-dibenzoyl-D-tartaric acid: (*S*)-2-amino-9,10-dimethoxy-1,6,7,11b-tetrahydro-4*H*-pyrido[2,1-a]isoquinoline-3-carboxylic acid ethyl ester, salt with (2*S*,3*S*)-bis-benzyloxy-succinic acid (**5a**)

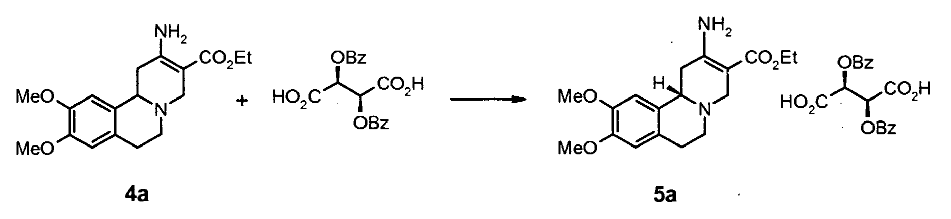

Classical resolution: A 500-ml four-necked flask equipped with a mechanical stirrer, reflux condenser, a thermometer, and an argon in/oulet was charged with racemic enamine **4a** (10.0 g, 30.1 mmol) and EtOH/H₂O 9:1 (125 ml) was added. The mixture was heated to 50 °C, whereupon a clear yellowish solution was obtained. (+)-O,O'-Dibenzoyl-D-tartaric acid (10.8 g, 30.1 mmol) was added in one portion to give a clear solution. After a couple of minutes, crystallization started. The mixture was allowed to slowly cool to ambient temperature over 2.5 h and was then stirred for another 14 hours. The suspension was filtered and the filter cake was washed with EtOH/H₂O (15 ml) at 0 °C. After drying under vacuum, (*S*)-enamine salt 5a (9.37 g, 45.1% yield, 98.0% ee) was obtained as white crystals. The enantiomeric excess was determined by HPLC on chiral stationary phase using a Chiralcel OD-H column.
mp = 161 °C

### Example 1b

A 1.5 L four-necked flask equipped with a mechanical stirrer, a reflux condenser, a Pt-100 thermometer and a nitrogen inlet was charged with 85.0 g (249 mmol) enamine 4a and 300 ml ethanol. Under stirring, 95.7 g (+)-O,O'-dibenzoyl-D-tartaric acid (262 mmol) were added as a solid, and the addition funnel was rinsed with 125 ml abs. ethanol. The white suspension was stirred for 24 h at 60°C, then allowed to slowly cool to RT. The slightly orange suspension was filtered, washed with its mother liquor for complete material transfer, then washed in several portions with totally 250 ml ethanol. The crystals were dried at 45°C / 10 mbar during 48 h, to give 154.6 g product (90% yield; assay: 97%; e.r. > 99:1) as a white powder.
mp = 161 °C

### Example 1c

Synthesis (*S*)-2-amino-9,10-dimethoxy-1,6,7,11b-tetrahydro-4*H*-pyrido[2,1-a]isoquinoline-3-carboxylic acid methyl ester, (*S*),(*S*)-2,3-bisbenzoyloxy-succinic acid (**5b**)

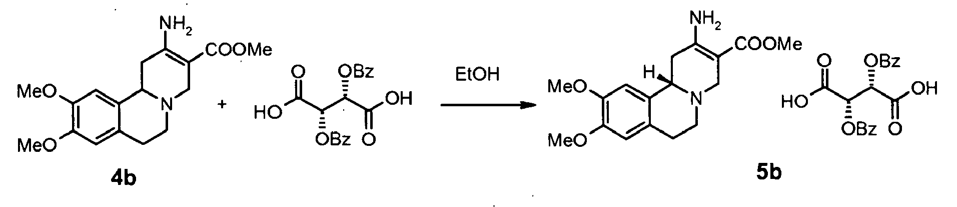

In a 10-ml two-necked round bottom flask equipped with a reflux condenser and an argon in/outlet, 500 mg of methyl ester **4b** and 597 mg of (+)-O,O'-dibenzoyl-D-tartaric acid were dissolved in 3 ml of EtOH at room temperature. The mixture was stirred in a 65 °C oil bath for 20 h. The resulting yellow suspension was cooled to room temperature over 1 h, diluted with 5 ml of EtOH and filtered. The crystals were washed twice with 5 ml of EtOH, and dried under high vacuum to give 643 mg (91.6% ee) of 5b as an off-white solid..

NMR (DMSO-d₆, 400 MHz): δ 8.00-7.95 (m, 4H); 7.70-7.66 (m, 2H); 7.57-7.53 (m, 4H), 6.81 (s, 1H); 6.62 (s, 1H); 5.73 (s, 2H); 3.91-3.65 (m, 8H); 3.60 (s, 3H); 3.35-3.20 (m, 2H); 3.15-3.03 (m, 1H); 3.00-2.89 (m, 1H), 2.80-2.60 (m, 2H); 2.40-2.28(m, 1H);

### Example 1d

Synthesis of (*S*)-2-amino-9,10-dimethoxy-1,6,7,11b-tetrahydro-4*H*-pyrido[2,1-a]isoquinoline-3-carboxylic acid isopropyl ester, (*S*),(*S*)-2,3-bisbenzoyloxy-succinic acid (5c)

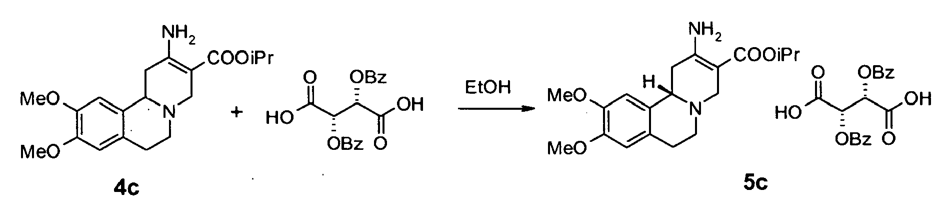

In a 10-ml two-necked round-bottom flask equipped with a reflux condenser and an argon in/outlet, 500 mg of isopropyl ester 4c and 549 mg of (+)-O,O'-dibenzoyl-D-tartaric acid were suspended in 3 ml of EtOH at room temperature. The mixture was stirred in a 65 °C oil bath for 20 h. The resulting suspension was cooled to room temperature over 2 h, diluted with 5 ml of EtOH, and filtered. The solid was washed twice with 5 ml of EtOH, dried under high vacuum to give 643 mg (> 99.5% ee) of 5c as an off-white solid.
mp=156 °C

### Example 2

Procedure for the optical resolution of (±)-enamine 1 using dibenzoyl-D-tartaric acid monodimethylamide: (*S*)-2-amino-9, 10-dimethoxy-1,6,7,11b-tetrahydro-4H-pyrido[2,1-a]isoquinoline-3-carboxylic acid ethyl ester, salt with (2*S*,3*S*)-bis-benzyloxy-*N,N*-dimethylsuccinamic acid (**5d**)

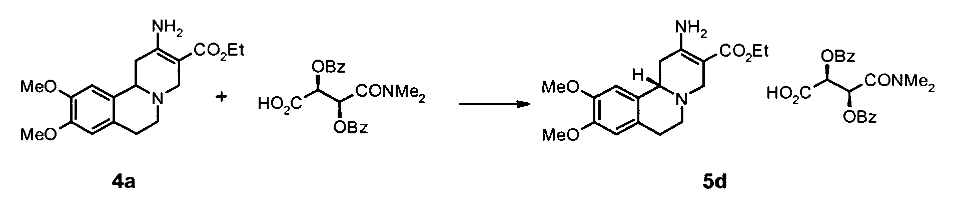

A 100-ml four-necked flask equipped with a mechanical stirrer, a reflux condenser, a thermometer, and an argon in/outlet was charged with racemic enamine **1** (10.0 g, 30.1 mmol) and EtOH (60 ml) was added. The mixture was heated to 50 °C, whereupon a clear solution was obtained. (+)-Dibenzoyl-D-tartaric acid monodimethylamide (11.5 g, 30.0 mmol) was added in one portion to give a clear yellowish solution. Five minutes after addition of the resolving agent, crystallization started at 50 °C. The mixture was allowed to slowly cool to ambient temperature and was stirred at this temperature for another 12 hours. The suspension was filtered and the filter cake was washed with EtOH (15 ml) at 0 °C. After drying under vacuum, (*S*)-enamine salt 3 (9.09 g, 42.1% yield, 99.3% ee) was obtained as white crystals. The enantiomeric excess was determined by HPLC on chiral stationary phase using a Chiralcel OD-H column.
mp = 161 °C

### Example 3

Procedure for the preparation of (*S*)-2-amino-9,10-dimethoxy-1,6,7,11b-tetrahydro-4H-pyrido[2,1-a] isoquinoline-3-carboxylic acid ethyl ester (**5e**)

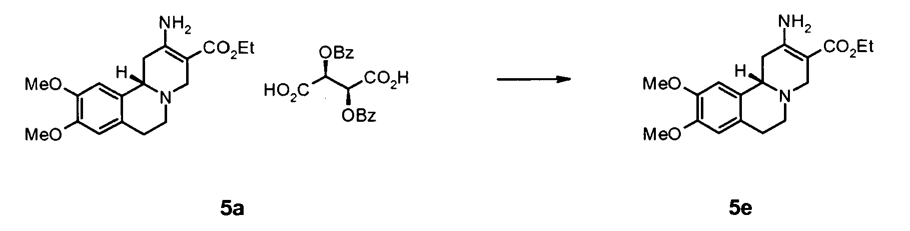

A 500-ml one-necked round bottom flask with a magnetic stirrer was charged with (*S*)-enamine tartaric acid salt **5a** (18.6 g, 29.9 mmol, 99.0% ee) and CH₂Cl₂ (180 ml). Sodium hydroxide solution (1.0 N, 180 ml) was added and the mixture stirred at room temperature for 5 minutes. The mixture was transferred to a separating funnel and the aqueous phase was extracted with CH₂Cl₂ (180 ml). Drying over Na₂SO₄, filtration and evaporation of the solvent gave the desired (*S*)-enamine **5e** (8.77 g, 98% yield, 99.0% ee) as a yellow foam. The enantiomeric excess was determined by HPLC on chiral stationary phase using a Chiralcel OD-H column.

### Example 4

Preparation of (2S,3S,11bS)- 2-tert-Butoxycarbonylamino-9,10-dimethoxy-1,3,4,6,7,11b-hexahydro-2H-pyrido[2,1-a]isoquinoline]-3-carboxylic acid ethyl ester (**6**)

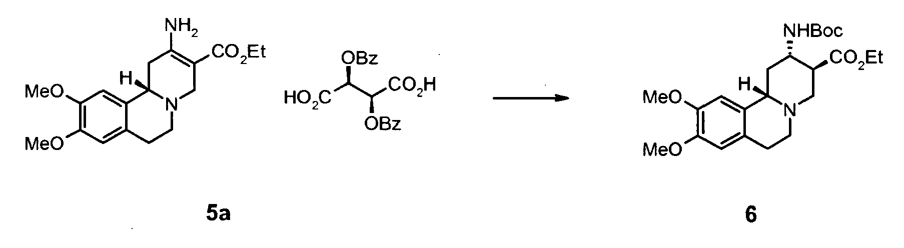

A 1.5 L four-necked flask equipped with a mechanical stirrer, a Pt-100 thermometer and a nitrogen inlet was charged with 250 g (362 mmol) of the tartaric acid salt 5a and 625 ml of dry THF. To the 10°C pre-cooled suspension cooled to 0-5 °C was slowly added 156 ml (1.99 mol) of trifluoro-acetic acid during 30 min, maintaining the temperature at 0-5°C. A yellow solution was obtained which was kept at a temperature of 0 - 5 °C.

In a second 1.5 L four-necked flask equipped with a mechanical stirrer, a Pt-100 thermometer, a reflux condenser and a nitrogen inlet, 14.27 g (362 mmol) of sodium borohydride and 375 ml of dry THF were charged. The resulting suspension was cooled to -10 to - 20 °C. To this suspension was slowly added the reaction mixture from the first flask during 30 min, maintaining the temperature at -10 to -20 °C. The addition initially was strongly exothermic and a vigorous hydrogen evolution took place.

After the addition, the reaction mixture was stirred at to -5 to 0 °C for 24 hours. After completion of the reduction, 1.25 L of water was then cautiously added, followed by 1.25 1 of dichloromethane: The acidic reaction mixture was then slowly basified using 325 ml of 32 % sodium hydroxide solution during ca. 40 min, until a pH of 13 -14 was achieved, maintaining the temperature at -5 to 0 °C.

The organic phase was separated, washed with 1.25 L of 10 % brine, followed by 1.25L of water. The aqueous phases were collected and extracted with 1.25 L of dichloromethane. The organic phases were collected and evaporated to dryness under reduced pressure at 45 °C.

The red orange residue was then taken up in 800 ml of dichloromethane and transferred to a 1.5 L four-necked flask equipped with a mechanical stirrer, a Pt-100 thermometer, a reflux condenser, a nitrogen inlet and a dropping funnel.

To the crude reaction mixture, a solution of 88.68 g of di-tert.-butyl dicarbonate in 200 ml of dichloromethane was added at RT. The reaction is slightly exothermic.

The reaction mixture was stirred overnight at RT. After completion of the reaction, the crude mixture was evaporated under reduced pressure at 45 °C up to a volume of 400 ml. The residual dichloromethane was evaporated through solvent exchange under constant volume with 1.51 of heptane.

The obtained crystals were suspended in 300 ml of heptane and stirred at room temperature for 3 hours. The crystals were filtered, washed portionwise with totally 625 ml of heptane and dried under reduced vacuum at 45 °C for 48 hours to give 101.6 g product (64% yield; assay: 99%) as a white powder.
MS: m/e 435 (M+H)⁺, 380, 379, 318.

### Example 5

Preparation of [(2S,3S,11bS)-(3-Carbamoyl-9,10-dimethoxy-1,3,4,6,7,11b-hexahydro-2H-pyrido[2,1-a]isoquinolin-2-yl)]-carbamic acid tert-butyl ester (7)

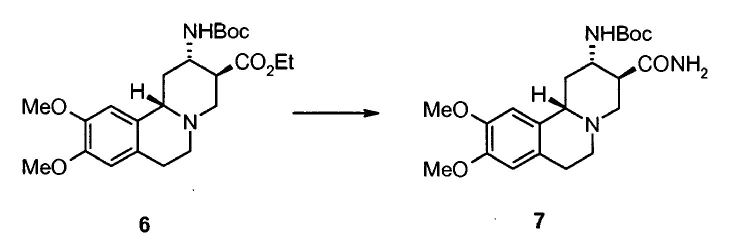

A 1 L four-necked flask equipped with a mechanical stirrer, a Pt-100 thermometer, a dropping funnel and a nitrogen inlet was charged with 50.0 g (113 mmol) of ester **6** and dissolved in 488 ml THF. To this red solution 22.6 ml (566 mmol) formamide was added at room temperature followed by 44.0 ml sodium methoxide (0.238 mol, 5.4 M) during 15 5 min. During the addition the suspension turned into a slightly reddish suspension. The mixture was stirred overnight at RT. It turned to a thick, but well stirrable white suspension which was diluted with 240 ml methanol and stirred for 20 min. The suspension was filtered off and washed portionwise with a mixture of 120 ml THF and 60 ml methanol. The crystals were dried at 40-45°C at ≤ 10 mbar for 24 hours, to give 43.0 g amide 7 (91.3% yield; assay: 97.5%).
MS: m/e 406 (M+H)⁺, 388, 351, 332, 255.

### Example 6

Direct Preparation of [(2S,3S,11bS)- (3-Carbamoyl-9,10-dimethoxy-1,3,4,6,7,11b-hexahydro-2H-pyrido[2,1-a]isoquinolin-2-yl)]-carbamic acid tert-butyl ester from the tartaric acid salt 5a (without isolation of ester **6**)

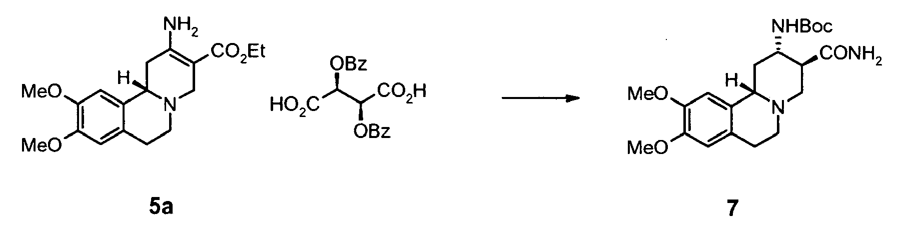

A four-necked flask equipped with a mechanical stirrer, a Pt-100 thermometer, a dropping funnel and a nitrogen inlet was charged with 4.05 g (103 mmol) sodium borohydride and 165 ml dry THF. The suspension was cooled to -30 to -20°C and treated at this temperature within one hour with 47.8 g (400 mmol) trifluoro acetic acid. To the resulting solution was added at -30 to -20°C in one portion 54.60 g (79 mmol) tartaric acid salt 5a. The mixture was allowed to warm to RT within 7 hours and then stirred at this temperature for additional 5 hours. The mixture was then added at 0 to 10°C to 200 ml water and the pH was adjusted to pH 7.5 to 8.0 by the addition of 43 g sodium hydroxide solution (28 % in water). Approximately 200 ml of THF were then distilled off under reduced pressure and replaced by the same amount of dichloromethane. The pH was adjusted to pH 11.0 to 11.5 by the addition of approximately 36 g sodium hydroxide solution (28 % in water). The stirrer was turned off and the layers were allowed to separate. The organic layer was separated and the aqueous layer was extracted with dichloromethane (1×90 ml). The combined organic layers were washed with water (1×90 ml) and then treated at RT within 10 minutes with a solution of 22.0 g (99 mmol) di-tert.-butyl dicarbonate in 44 ml dichloromethane. After 2 hours at RT dichloromethane was distilled off and continuously replaced by 900 ml THF. The mixture (approximately 400 ml) was then successively treated at 32 to 38°C with 35.65 g (788 mmol) formamide and 42.7 g (237 mmol) sodium methoxide solution (30 % in methanol) and the resulting suspension was stirred at 32 to 38°C for 10 hours. The mixture was treated with 180 ml water and heated at 60 to 65°C for 4 hours. The suspension was cooled to RT within 1 to 2 hours and stirred at RT for 1 hour. The crystals were filtered off, washed in two portions with a mixture of 90 ml THF and 45 ml water and dried at 60 to 70°C at ≤ 30 mbar for 15 hours, to afford 25.40 g of amide 7 (79.2% yield; assay: 98.7%(m/m)).
MS: m/e 406 (M+H)⁺, 388, 351, 332, 255.
mp: 270°C (DSC; slight decomp. at > 250°C)

### Example 7a

Preparation of (2S,3S,11bS)-(3-Amino-9,10-dimethoxy-1,3,4,6,7,11b-hexahydro-2H-pyrido[2,1-a]isoquinolin-2-yl)]-carbamic acid tert-butyl ester (**8**)

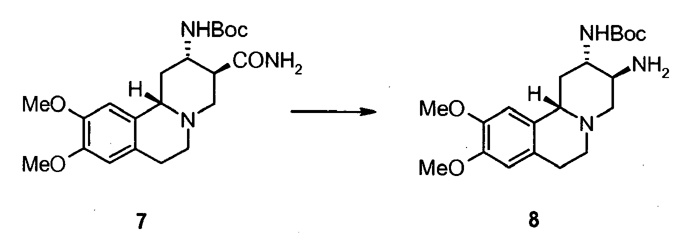

A 6 L four-necked flask equipped with a mechanical stirrer, a Pt-100 thermometer, a dropping funnel and a nitrogen inlet was charged with 100 g (242 mmol) amide 7. 982 ml 2 N sodium hydroxide solution were added and the mixture stirred for 5 minutes at RT. 1.75 L acetonitrile were added and stirring was continued for an additional 30 min. To the resulting suspension was added a solution of 95.5 g (291 mmol) diacetoxyiodosobenzene in 240 ml water and 500 ml acetonitrile during 15 min, maintaining the temperature at 18-22°C. The slightly yellow reaction mixture was stirred at RT for 15 min. A slightly yellow two-phase mixture containing some undissolved crystals was formed, to which 400 g sodium chloride were added and the mixture was further stirred for 20 minutes at RT, then cooled to 5 °C. A solution of 220 ml 25 % hydrochloric acid and 220 ml water were slowly added during 30 min to bring the pH to about 5.5. From pH of 8 on, a precipitate formed. The suspension was further stirred for 75 minutes at 5 to 10°C and pH 5.5. The suspension was filtered off, transferred back into the reactor and suspended in 1.5 L dichloromethane. 1 L of a 10 % sodium bicarbonate solution was added to the suspension and the mixture was stirred for 15 minutes, whereas pH 8 was reached. The organic phase was separated and the aqueous phase was extracted again with 1 L dichloromethane. The organic phases were collected and concentrated at 45 °C to just before the crystallization point. 275 ml TBME were added and the resulting suspension stirred for 1 hour at RT and then for 1.5 hour at 0 to 4 °C. The crystals were then filtered off and washed portionwise with totally 150 ml of cold TBME.

The crystals were dried at 40-45°C at 10 mbar for 48 hours, then suspended in a mixture of 530 ml ethanol and 530 ml methanol and stirred for 2 hours at RT. The precipitate was filtered off and washed portionwise with totally 100 ml of a 1:1 mixture of methanol and ethanol. The filtrate was evaporated to dryness at 50 °C and the crystals dried at 50°C / 1 mbar. They were then suspended in 400 ml TBME, stirred for 2 hours at 20 °C and then for 2 hours at 0 °C. The crystals were filtered off and washed portionwise with totally 200 ml cold TBME. The crystals were dried at 40-45°C. at ≤ 20 mbar for 24 hours to give 67.2 g amine **8** (73% yield; assay: 99%)
MS: m/e 378 (M+H)⁺, 322, 306, 305.

### Example 7b

Preparation of (2S,3S,11bS)-(3-Amino-9,10-dimethoxy-1,3,4,6,7,11b-hexahydro-2H-pyrido[2,1-a]isoquinolin-2-yl)]-carbamic acid tert-butyl ester (**8**)

A 1 L four-necked flask equipped with a mechanical stirrer, a Pt-100 thermometer, a dropping funnel and a nitrogen inlet was charged with 20.00 g (49 mmol) amide 7, 85 ml water and 215 ml acetonitrile. The suspension was treated at 5 to 10°C within 30 minutes with 69.7 g sodium hydroxide solution (28 % in water). The mixture was heated to 15 to 20°C and treated at this temperature within 2 to 3 hours with a solution of 18.07 g (56 mmol) iodosobenzene diacetate in 46 ml water and 97 ml acetonitrile. The mixture was stirred at 15 to 20°C for 30 minutes, concentrated under reduced pressure to a residual volume of approximately 250 ml and then treated at RT with 30 g hydrochloric acid (37% in water) to adjust the pH to 9.4 to 9.7. Dichloromethane (200 ml) was added and the layers were allowed to separate. The organic layer was separated and the aqueous layer was extracted with dichloromethane (1×80 ml). To remove insoluble urea by-products the combined organic layers were filtered. From the filtrate dichloromethane was distilled off and continuously replaced by 220 ml toluene. The suspension was heated to 70°C and the resulting slightly turbid solution was polish filtered. The filtrate was concentrated under reduced pressure to a residual volume of approximately 150 ml. The resulting suspension was heated to 75 to 85°C and stirred at this temperature until a clear solution was obtained. The solution was then allowed to cool to 0 to 5°C within 2 hours, whereby crystallization occurred at approximately 60°C. After 2 hours at 0 to 5°C the crystals were filtered off, washed in two portions with 50 ml toluene and dried at 45 to 55°C at ≤ 30 mbar for 15 hours, to afford 14.70 g of amine **8** (79.0% yield; assay: 99.6%(m/m)).
MS: m/e 378 (M+H)⁺, 322, 306, 305.
mp:170°C (DSC)

### Example 8

Transformation of (2S,3S,11bS)-(3-Amino-9,10-dimethoxy-1,3,4,6,7,11b-hexahydro-2H-pyrido[2,1-a]isoquinolin-2-yl)]-carbamic acid tert-butyl ester into (*S*)-1-((2*S*,3*S*,11*bS*)-2-amino-9,10-dimethoxy-1,3,4,6,7,11b-hexahydro-2H-pyrido[2,1-a] isoquinolin-3-yl)-4-fluoromethyl-pyrrolidin-2-one.

### a) Preparation of 4-fluoromethyl-5H-furan-2-one

A 6 L reactor equipped with a mechanical stirrer, a Pt-100 thermometer, a dropping funnel and a nitrogen inlet was charged with 500 g (4.38 mmol) 4-hydroxymethyl-5H-furan-2-one and 2.0 L dichloromethane. The solution was cooled to -10 °C and 1.12 kg (4.82 mol) bis-(2-methoxyethyl)aminosulfur trifluoride (Deoxo-Fluor) was added during 50 min, maintaining the temperature at -5 to -10 °C with a cooling bath. During the addition a yellowish emulsion formed, which dissolved to an orange-red solution after completed addition. This solution was stirred for 1.5 h at 15-20 °C, then cooled to -10 °C. A solution of 250 ml water in 1.00 L ethanol was added during 30 min, maintaining the temperature between -5 and -10 °C, before the mixture was allowed to reach 15 - 20 °C. It was then concentrated in a rotatory evaporator to a volume of ca. 1.6 L at 40 °C / 600-120 mbar. The residue was dissolved in 2.0 L dichloromethane and washed three times with 4.0 L 1N hydrochloric acid. The combined aqueous layers were extracted three times with 1.4 L dichloromethane. The combined organic layers were evaporated in a rotatory evaporator to give 681 g crude product as a dark brown liquid. This material was distilled over a Vigreux column at 0.1 mbar, the product fractions being collected between 71 and 75 °C (312 g). This material was re-distilled under the same conditions, the fractions being collected between 65 and 73 °C, to give 299 g 4-fluoromethyl-5H-furan-2-one (58% yield; assay: 99%).
MS: m/e 118 M⁺, 74, 59, 41.

### b) Preparation of (S)-4-fluoromethyl-dihydro-furan-2-one

A 2 L autoclave equipped with a mechanical stirrer was charged with a solution of 96.0 g 4-fluoromethyl-5H-furan-2-one (8.27 x 10-1 mol) in 284 mL methanol. The autoclave was sealed and pressurized several times with argon (7 bar) in order to remove any traces of oxygen. At ~1 bar argon, a solution of 82.74 mg Ru(OAc)₂((R)-3,5-tBu-MeOBIPHEP) (6.62 x 10-5 mol) (S/C 12500) in 100 mL methanol was added under stirring from a catalyst addition device previously charged in a glove box (O₂ content < 2 ppm) and pressurized with argon (7 bar). The argon atmosphere in the autoclave was replaced by hydrogen (5 bar). At this pressure, the reaction mixture was stirred (~800 rpm) for 20 h at 30 °C and then removed from the autoclave and concentrated *in vacuo*. The residue was distilled to afford 91.8 g (94%) (*S*)-4-fluoromethyl-dihydro-furan-2-one. The chemical purity of the product was 99.7% by GC-area.

### c) Preparation of (2S,3S,11bS)-3-(3-Fluoromethyl-4-hydroxy-butyrylamino)-9,10-dimethoxy-1,3,4,6,7,11b-hexahydro-2H-pyrido[2,1-a]isoquinolin-2-yl]-carbamic acid tert-butyl ester

A 1.5 L reactor equipped with a mechanical stirrer, a Pt-100 thermometer, a dropping funnel and a nitrogen inlet was charged with 50 g (128 mmol) (2*S*,3*S*,11*bS*)-3-amino-9,10-dimethoxy-1,3,4,6,7,11b-hexahydro-2H-pyrido[2,1-a]isoquinolin-2-yl)-carbamic acid tert-butyl ester , 500 mL toluene and 2.51 g (25.6 mmol) 2-hydroxypyridine. To this slightly brownish suspension, 22.7 g (192 mmol) of (*S*)-4-fluoromethyl-dihydro-furan-2-one was added dropwise at RT. No exothermy was observed during the addition. The dropping funnel was rinsed portionwise with totally 100 mL toluene. The suspension was heated to reflux, whereas it turned into a clear solution starting from 60 °C, after 40 min under reflux a suspension formed again. After totally 23 h under reflux, the thick suspension was cooled to RT, diluted with 100 mL dichloromethane and stirred for 30 min at RT. After filtration, the filter cake was washed portionwise with totally 200 mL toluene, then portionwise with totally 100 mL dichloromethane. The filter cake was dried at 50 °C / 10 mbar for 20 h, to give 60.0 g product (94% yield; assay: 100%).
MS: m/e 496 (M+H)⁺, 437.

### d) Preparation of (2S,3S,11bS)-3-((4S)-Fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester

A 1.5 L reactor equipped with a mechanical stirrer, a Pt-100 thermometer, a dropping funnel, a cooling bath and a nitrogen inlet was charged with 28 g (56.5 mmol) of (2S,3S,11bS)-3-(3-fluoromethyl-4-hydroxy-butyrylamino)-9,10-dimethoxy-1,3,4,6,7,11b-hexahydro-2H-pyrido[2,1-a]isoquinolin-2-yl]-carbamic acid tert-butyl ester and 750 mL THF. The mixture was cooled to 0 °C and a solution of 6.17 mL (79 mmol) methanesulfonic acid in 42 mL THF was added during 10 min, maintaining the temperature at 0-5 °C. At 0 °C a solution of 12.6 mL (90.2 mmol) triethylamine in 42 mL THF was added during 15 min. The resulting suspension was stirred for 80 min at 0-5 °C, whereas it became gradually thicker. Then 141 mL (141 mmol) 1 M lithiumbis(trimethylsilyl)amide were added to the mixture during 15 min, whereas the suspension dissolved. The solution was allowed to reach RT during 60 min under stirring. 500 mL water was added without cooling, the mixture was extracted and the aqueous phase was subsequently extracted with 500 mL and 250 mL dichloromethane. The organic layers were each washed with 300 mL half saturated brine, combined and evaporated on a rotatory evaporator. The resulting foam was dissolved in 155 mL dichloromethane, filtered and again evaporated to give 30.5 g crude product as a slightly brownish foam. This material was dissolved in 122 mL methanol, resulting in a thick suspension, which dissolved on heating to reflux. After 20 min of reflux the solution was allowed to gradually cool to RT during 2 h, whereas crystallization started after 10 min. After 2 h the suspension was cooled to 0 °C for 1 h, followed by -25°C for 1 h. The crystals were filtered off via a pre-cooled glasssinter funnel, washed portionwise with 78 mL TBME and dried for 18 h at 45 °C / 20 mbar, to give 21.0 g of the title product as white crystals (77% yield; assay: 99.5%).
MS: m/e 478 (M+H)⁺, 437, 422.

### e) Preparation of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride

A 2.5 L reactor equipped with a mechanical stirrer, a Pt-100 thermometer, a dropping funnel and a nitrogen inlet was charged with 619 g (1.30 mol) of (2*S*,3*S*,11*bS*)-3-((4S)-fluoromethyl-2-oxo-pyrrolidin-l-yl)-9,10-dimethoxy-1,3,4,6,7,11b-hexahydro-2H-pyrido[2,1-a]isoquinolin-2-yl]-carbamic acid tert-butyl ester, 4.2 L isopropanol and 62 mL water and the suspension was heated to 40-45 °C. In a second vessel, 1.98 L isopropanol was cooled to 0 °C and 461 mL (6.50 mol) acetyl chloride was added during 35 min, maintaining the temperature at 0-7 °C. After completed addition, the mixture was allowed to reach ca. 15 °C and was then slowly added to the first vessel during 1.5 h. After completed addition the mixture was stirred for 18 h at 40-45 °C, whereas crystallization started after 1 h. The white suspension was cooled to 20 °C during 2 h, stirred at that temperature for 1.5 h and filtered. The crystals were washed portionwise with 1.1 L isopropanol and dried for 72 h at 45°C / 20 mbar, to give 583 g of the product as white crystals (100% yield; assay: 99.0%)

## Claims

1. Process for the preparation of pyrido[2,1-a]isoquinoline derivatives of the formula 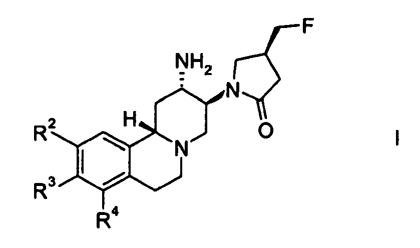 wherein R², R³ and R⁴ are each independently selected from the group consisting of hydrogen, halogen, hydroxy, C₁-C₆-alkyl, C₁-C₆-alkoxy and C₁-C₆-alkenyl, wherein C₁-C₆-alkyl, C₁-C₆-alkoxy and C₁-C₆-alkenyl may optionally be substituted by a group selected from C₁-C₆-alkoxycarbonyl, aryl and heterocyclyl,
comprising one or more of the steps a), b), c) or d), wherein
step a) comprises the optical resolution of an enamine of the formula 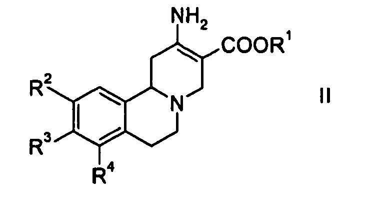 wherein R², R³ and R⁴ are as defined above and R¹ is C₁-C₆-alkyl or benzyl, in the presence of an optical active resolving agent to form the (S)-enamine salt of the formula 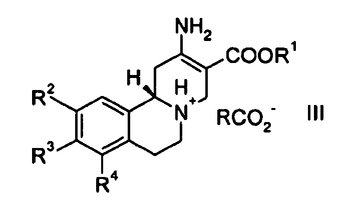 wherein R¹, R², R³ and R⁴ are as defined above and RCO₂⁻ is the conjugate base of the resolving agent;
step b) comprises the transformation of the (S)-enamine salt of formula III into the ester of formula 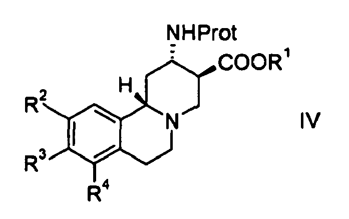 wherein R¹, R², R³ and R⁴ are as defined above and Prot stands for an amino protecting group;
step c) comprises amidation of the ester of formula IV to form the amide of formula 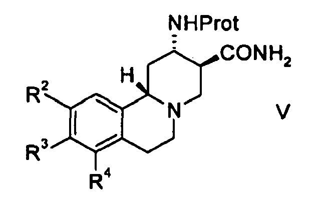 wherein R², R³, R⁴ and Prot are as defined above;
step d) comprises degradation of the amide of formula V to form the amine of formula 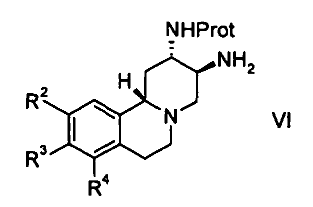 wherein R², R³, R⁴ and Prot are as defined above;
wherein the process comprises step a); and
wherein the process is **characterized in that** the optical resolution in step a) is a crystallization-induced dynamic resolution.

2. Process according to claim 1, comprising the steps a) and b).

3. Process according to claim 1, comprising the steps a) to d).

4. Process according to claim 1, wherein the steps b) and c) are carried out without isolation of intermediate IV.

5. Process according to claims 1 to 4, **characterized in that** the optical resolution in step a) is performed with a resolving agent of the formula 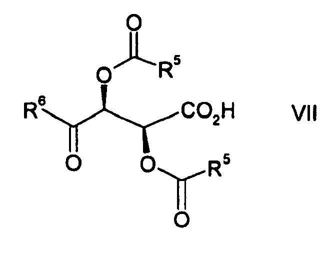 wherein
R⁵ is selected from the group consisting of unsubstituted phenyl, phenyl substituted by one, two, or three groups independently selected from C₁-C₆-alkyl, C₁-C₆-alkoxy and halogen, C₁-C₆-alkyl, benzyl, wherein the phenyl ring is unsubstituted or substituted by one, two, or three groups independently selected from C₁-C₆-alkyl, C₁-C₆-alkoxy and halogen, and -NH-phenyl, wherein the phenyl ring is unsubstituted or substituted by one, two, or three groups independently selected from C₁-C₆-alkyl, C₁-C₆-alkoxy and halogen; and
R⁶ is selected from the group consisting of hydroxy, C₁-C₆-alkoxy and -NR⁷R⁸, wherein R⁷ and R⁸ independently from each other are C₁-C₆-alkyl.

6. Process according to claims 1 to 5, **characterized in that** the optical resolution in step a) is performed with a resolving agent of the formula 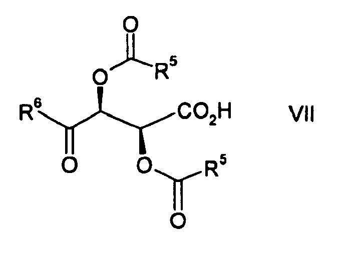 wherein R⁵ is selected from the group consisting of unsubstituted phenyl, phenyl substituted by one, two, or three groups independently selected from C₁-C₆-alkyl, C₁-C₆-alkoxy and halogen, and -NH-phenyl, wherein the phenyl ring is unsubstituted or substituted by one, two, or three groups independently selected from C₁-C₆-alkyl, C₁-C₆-alkoxy and halogen, and
R⁶ is hydroxy or -NR⁷R⁸, wherein R⁷ and R⁸ independently from each other are C₁-C₆-alkyl.

7. Process according to claims 1 to 6, **characterized in that** the optical resolution in step a) is performed with a resolving agent selected from (+)-O,O'-dibenzoyl-D-tartaric acid and (+)-O,O'-dibenzoyl-D-tartaric acid mono dimethylamide.

8. Process according to claim 1 to 7, **characterized in that** the optical resolution in step a) is performed in a solvent selected from the group consisting of water, methanol, ethanol, isopropanol, acetone, tetrahydrofuran, ethyl acetate, toluene and mixtures thereof.

9. Process according to claims 1 to 8, **characterized in that** the optical resolution in step a) is performed with the enamine of formula II wherein R¹ is methyl, ethyl or isopropyl.

10. Process according to claim 1, **characterized in that** the transformation of the (S)-enamine salt of formula III in step b) is performed by a reduction under acidic conditions followed by the introduction of an amino protecting group.

11. Process according to claim 10, **characterized in that** the reduction is performed with reducing agents selected from sodium borohydride, lithium borohydride and sodium cyanoborohydride.

12. Process according to claims 1, 10 or 11, **characterized in that** the reduction is performed in an organic solvent at temperatures of -40 °C to 30 °C.

13. Process according to claims 1, 10, 11 or 12, **characterized in that** an amino protecting group selected from the group consisting of trichloroethoxycarbonyl, benzyloxycarbonyl, chloroacetyl, trifluoroacetyl, phenylacetyl, formyl, acetyl, benzoyl, tert-butoxycarbonyl, para-methoxybenzyloxycarbonyl, diphenylmethoxycarbonyl, phthaloyl, succinyl, benzyl, diphenylmethyl, triphenylmethyl, methanesulfonyl, para-toluenesulfonyl, pivaloyl, trimethylsilyl, triethylsilyl and triphenylsilyl is introduced.

14. Process according to claim 1, **characterized in that** the amidation in step c) is performed with formamide/ sodium methoxide, formamide/ sodium ethoxide, acetamide/ sodium methoxide or acetamide/ sodium ethoxide.

15. Process according to claim 1 or 14, **characterized in that** the amidation in step c) is performed in an organic solvent at temperatures in the range of 10 °C to 70 °C.

16. Process according to claim 1, **characterized in that** the degradation of the amide of formula V in step d) is performed according to the principle of the Hofmann-degradation.

17. Process according to claims 1 or 16, **characterized in that** the degradation of the amide of formula V in step d) is performed with an oxidizing agent selected from iodosobenzene diacetate, iodosobenzene bistrifluoroacetate and iodosobenzene bistrichloroacetate.

18. Process according to claims 1 to 17 for the preparation of (2S,3S,11bS)-(3-amino-9,10-dimethoxy-1,3,4,6,7,11b-hexahydro-2H-pyrido[2,1-a]isoquinolin-2-yl)]-carbamic acid tert-butyl ester.

19. Process according to claims 1 to 17 for the preparation of (*S*)-1-((2*S*,3*S*,11*bS*)-2-amino-9,10-dimethoxy-1,3,4,6,7,11b-hexahydro-2H-pyrido[2,1-a] isoquinolin-3-yl)-4-fluoromethyl-pyrrolidin-2-one.

20. Process according to claims 1 to 17 for the preparation of (S)-1-((2S,3S,11bS)-2-amino-9,10-dimethoxy-1,3,4,6,7,11b-hexahydro-2H-pyrido[2,1-a]isoquinolin-3-yl)-4-fluoromethyl-pyrrolidin-2-one, comprising the process according to claims 1 to 17, followed by
e) coupling of (2*S*,3*S*,11*bS*)-3-amino-9,10-dimethoxy-1,3,4,6,7,11b-hexahydro-2H-pyrido[2,1-a]isoquinolin-2-yl)-carbamic acid tert-butyl ester with the (S)-4-fluoromethyldihydro-furan-2-one of formula 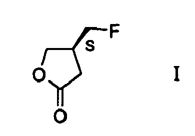
f) cyclization of the obtained (2*S*,3*S*,11*bS*)-3-(3-fluoromethyl-4-hydroxy-butyrylamino)-9,10-dimethoxy-1,3,4,6,7,11b-hexahydro-2H-pyrido[2,1-a]isoquinolin-2-yl]-carbamic acid tert-butyl ester in the presence of a base, and
g) deprotecting the obtained (2*S*,3*S*,11*bS*)-3-(4*S*)-tluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester.

21. (S)-Enamine salt of the formula 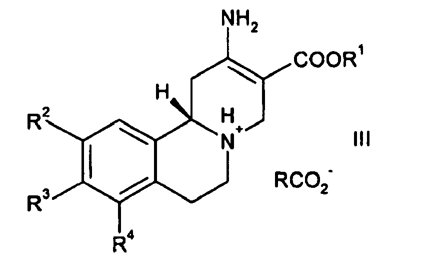 wherein R¹, R², R³ and R⁴ are as defined in claim 1 and RCO₂⁻ is the conjugate base of the resolving agent.

22. Ester of the formula 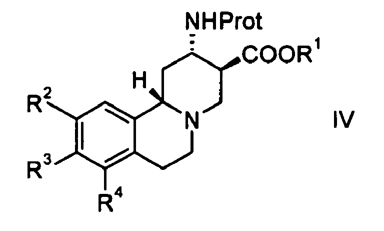 wherein R¹, R², R³ and R⁴ are as defined in claim 1 and Prot stands for an amino protecting group.

## Patentansprüche

1. Verfahren zur Herstellung von Pyrido[2,1-a]isochinolinderivaten der Formel 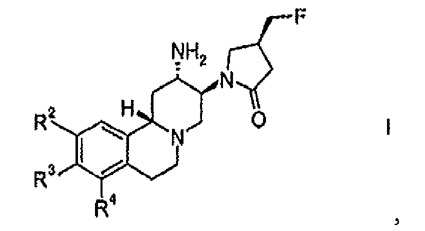 wobei R², R³ und R⁴ jeweils unabhängig voneinander aus der Gruppe ausgewählt sind, bestehend aus Wasserstoff, Halogen, Hydroxy, C₁-C₆-Alkyl, C₁-C₆-Alkoxy und C₁-C₆-Alkenyl, wobei C₁-C₆-Alkyl, C₁-C₆-Alkoxy und C₁-C₆-Alkenyl gegebenenfalls substituiert ist durch eine Gruppe, ausgewählt aus C₁-C₆-Alkoxycarbonyl, Aryl und Heterocyclyl,
umfassend einen oder mehrere der Schritte a), b), c) oder d), wobei
Schritt a) die optische Trennung eines Enamins der Formel 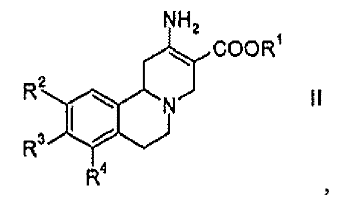 wobei R², R³ und R⁴ wie oben definiert sind und R¹ C₁-C₆-Alkyl oder Benzyl ist, in Gegenwart eines optisch aktiven Trennmittels unter Bildung des (S)-Enaminsalzes der Formel 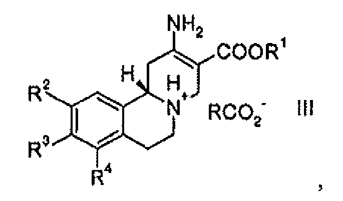 wobei R¹, R², R³ und R⁴ oben definiert sind und RCO₂⁻ die korrespondierende Base des Trennmittels ist, umfasst;
Schritt b) die Umwandlung des (S)-Enaminsalzes der Formel III in den Ester der Formel 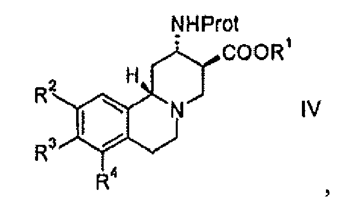 wobei R¹, R², R³ und R⁴ wie oben definiert sind und Prot für eine Aminoschutzgruppe steht, umfasst;
Schritt c) die Amidierung des Esters der Formel IV unter Bildung des Amids der Formel 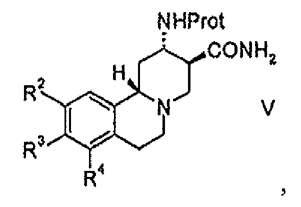 wobei R², R³, R⁴ und Prot wie oben definiert sind, umfasst,
Schritt d) den Abbau des Amids der Formel V unter Bildung des Amins der Formel 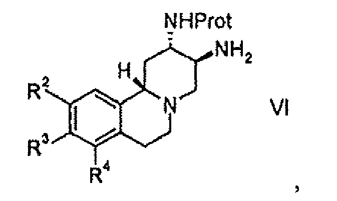 wobei R², R³, R⁴ und Prot wie oben definiert sind, umfasst und
wobei das Verfahren Schritt a) umfasst und
wobei das Verfahren **dadurch gekennzeichnet ist, dass** die optische Trennung in Schritt a) eine Kristallisations-induzierte dynamische Trennung ist.

2. Verfahren nach Anspruch 1, umfassend die Schritte a) und b).

3. Verfahren nach Anspruch 1, umfassend die Schritte a) bis d).

4. Verfahren nach Anspruch 1, wobei die Schritte b) und c) ohne Isolation des Zwischenproduktes IV durchgeführt werden.

5. Verfahren nach den Ansprüchen 1 bis 4, **dadurch gekennzeichnet, dass** die optische Trennung in Schritt a) mit einem Trennmittel der Formel 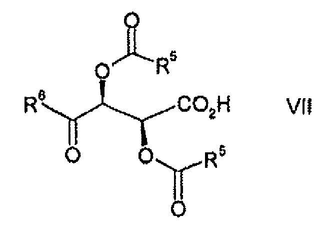 durchgerührt wird, wobei
R⁵ aus der Gruppe ausgewählt ist, bestehend aus unsubstituiertem Phenyl, Phenyl, substituiert durch eine, zwei oder drei Gruppen, unabhängig voneinander aus- gewählt aus C₁-C₆-Alkyl, C₁-C₆-Alkoxy und Halogen, C₁-C₆-Alkyl, Benzyl, wobei der Phenylring unsubstituiert oder substituiert ist durch eine, zwei oder drei Gruppen, unabhängig voneinander ausgewählt aus C₁-C₆-Alkyl, C₁-C₆-Alkoxy und Halogen, und -NH-Phenyl, wobei der Phenylring unsubstituiert oder substituiert ist durch eine, zwei oder drei Gruppen, unabhängig voneinander ausgewählt aus C₁-C₆-Alkyl, C₁-C₆-Alk- oxy und Halogen; und
R⁶ aus der Gruppe ausgewählt ist, bestehend aus Hydroxy, C₁-C₆-Alkoxy und -NR⁷R⁸, wobei R⁷ und R⁸ unabhängig voneinander C₁-C₆-Alkyl sind.

6. Verfahren nach den Ansprüchen 1 bis 5, **dadurch gekennzeichnet, dass** die optische Trennung in Schritt a) mit einem Trennmittel der Formel 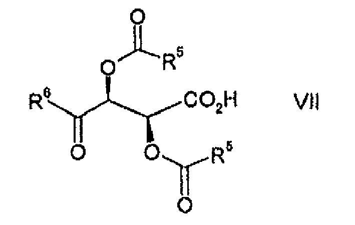 durchgeführt wird, wobei
R⁵ aus der Gruppe ausgewählt ist, bestehend aus unsubstituiertem Phenyl, Phenyl, substituiert durch eine, zwei oder drei Gruppen, unabhängig voneinander ausgewählt aus C₁-C₆-Alkyl, C₁-C₆-Alkoxy und Halogen, und -NH-Phenyl, wobei der Phenylring unsubstituiert oder substituiert ist durch eine, zwei oder drei Gruppen, unabhängig voneinander ausgewählt aus C₁-C₆-Alkyl, C₁-C₆-Alkoxy und Halogen, und
R⁶ Hydroxy oder -NR⁷R⁸ ist, wobei R⁷ und R⁸ unabhängig voneinander C₁-C₆-Alkyl sind.

7. Verfahren nach den Ansprüchen 1 bis 6, **dadurch gekennzeichnet, dass** die optische Trennung in Schritt a) mit einem Trennmittel, ausgewählt aus (+)-O,O'-Dibenzoyl-D-weinsäure und (+)-O,O'-Dibenzoyl-D-weinsäuremonadimethylamid, durchgeführt wird.

8. Verfahren nach den Ansprüchen 1 bis 7, **dadurch gekennzeichnet, dass** die optische Trennung in Schritt a) in einem Lösungsmittel, ausgewählt aus der Gruppe, bestehend aus Wasser, Methanol, Ethanol, Isopropanol, Aceton, Tetrahydrofuran, Ethylacetat, Toluol und Gemischen davon, durchgeführt wird.

9. Verfahren nach den Ansprüchen 1 bis 8, **dadurch gekennzeichnet, dass** die optische Trennung in Schritt a) mit dem Enamin der Formel II, wobei R¹ Methyl, Ethyl oder Isopropyl ist, durchgeführt wird.

10. Verfahren nach Anspruch 1, **dadurch gekennzeichnet, dass** die Umwandlung des (S)-Enaminsalzes der Formel III in Schritt b) durch eine Reduktion unter sauren Bedingungen, gefolgt von der Einführung einer Aminoschutzgruppe, durchgeführt wird.

11. Verfahren nach Anspruch 10, **dadurch gekennzeichnet, dass** die Reduktion mit Reduktionsmitteln, ausgewählt aus Natriumborhydrid, Lithiumborhydrid und Natriumcyanoborhydrid, durchgeführt wird.

12. Verfahren nach den Ansprüchen 1, 10 oder 11, **dadurch gekennzeichnet, dass** die Reduktion in einem organischen Lösungsmittel bei Temperaturen von -40 °C bis 30 °C durchgeführt wird.

13. Verfahren nach den Ansprüchen 1, 10, 11 oder 12, **dadurch gekennzeichnet, dass** eine Aminoschutzgruppe, ausgewählt aus der Gruppe, bestehend aus Trichlorethoxycarbonyl, Benzyloxycarbonyl, Chloracetyl, Trifluoracetyl, Phenylacetyl, Formyl, Acetyl, Benzoyl, tert-Butoxycarbonyl, para-Methoxybenzyloxycarbonyl, Diphenylmethoxycarbonyl, Phthaloyl, Succinyl, Benzyl, Diphenylmethyl, Triphenylmethyl, Methansulfonyl, para-Toluolsulfonyl, Pivaloyl, Trimethylsilyl, Triethylsilyl und Triphenylsilyl, eingeführt wird.

14. Verfahren nach Anspruch 1, **dadurch gekennzeichnet, dass** die Amidierung in Schritt c) mit Formamid/Natriummethoxid, Formamid/Natriumethoxid, Acetamid/Natriurnmethoxid oder Acetamid/Natriumethoxid durchgeführt wird.

15. Verfahren nach Anspruch 1 oder 14, **dadurch gekennzeichnet, dass** die Amidierung in Schritt c) in einem organischen Lösungsmittel bei Temperaturen im Bereich von 10 °C bis 70 °C durchgeführt wird.

16. Verfahren nach Anspruch 1, **dadurch gekennzeichnet, dass** der Abbau des Amids der Formel V in Schritt d) gemäß dem Prinzip des Hofmann-Abbaus durchgeführt.

17. Verfahren nach den Ansprüchen 1 oder 16, **dadurch gekennzeichnet, dass** der Abbau des Amids der Formel V in Schritt d) mit einem Oxidationsmittel, ausgewählt aus Iodosobenzoldiacetat, Iodosabenzolbistrifluoracetat und Iodosobenzolbistriehloracetat, durchgeführt wird.

18. Verfahren nach den Ansprüchen 1 bis 17 zur Herstellung von (2S,3S,11bS)-(3-Amino-9,10-dimethoxy-1,3,4,6,7,11b-hexahydro-2H-pyrido[2,1-a]isochinolin-2-yl)]-carbamidsäure-tert-butylester.

19. Verfahren nach den Ansprüchen 1 bis 17 zur Herstellung von (S)-1-((2S,3S,11*b*S)-2-Amino-9,10-dimethoxy-1,3,4,6,7,11b-hexahydro-2H-pyrido[2,1-a]isochinolin-3-yl)-4-fluormethyl-pyrrolidin-2-on.

20. Verfahren nach den Ansprüchen 1 bis 17 zur Herstellung von (S)-1-((2S,3S,11bS)-2-Amino-9,10-dimethoxy-1,3,4,6,7,11b-hexahydro-2H-pyrido[2,1-a]isochinolin-3-yl)-4-fluormethyl-pyrrolidin-2-on, umfassend das Verfahren nach den Ansprüchen 1 bis 17, gefolgt von
e) Koppeln von (2S,3S,11*b*S)-3-Amino-9,10-dimethoxy-1,3,4,6,7,11b-hexahydro-2H-pyrido-[2,1-a]isochinolin-2-yl)-carbamidsäure-tert-butylester mit dem (S)-4-Fluormethyl-dihydrofuran-2-on der Formel 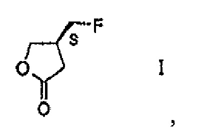
f) Cyclisieren des erhaltenen (2S,3S,11bS)-3-(3-Fluormethyl-4-hydroxy-butyrylamino)-9,10-dimethoxy-1,3,4,6,7,11b-hexahydro-2H-pyrido[2,1-a]isochinlin-2-yl]-carbamidsäure-tert-butylesters in Gegenwart einer Base und
g) Entschützen des erhaltenen (2S,3S,11*b*S)-3-((4S)-Fluormethyl-2-oxo-pyrrolidin-1-yl)-9,10-dimethoxy-1,3,4,6,7,11b-hexahydro-2H-pyrido[2,1-a]isochinolin-2-yl]-carbamidsäure-tert-butylesters.

21. (S)-Enaminsalz der Formel 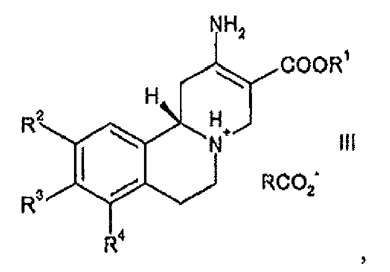 wobei R¹, R², R³ und R⁴ wie in Anspruch 1 definiert sind und RCO₂⁻ die korrespondierende Base des Trennmittels ist.

22. Ester der Formel 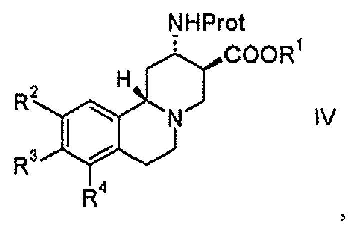 wobei R¹, R², R³ und R⁴ wie in Anspruch 1 definiert sind und Prot für eine Aminoschutzgruppe steht.

## Revendications

1. Procédé pour la préparation de dérivés de pyrido[2,1-a]isoquinoléine de formule 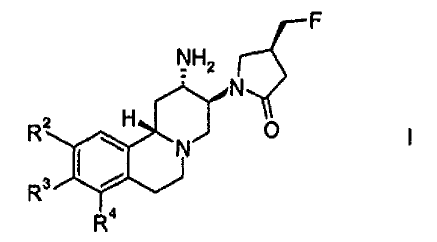 dans laquelle R², R³ et R⁴ sont choisis chacun indépendamment dans le groupe consistant en un atome d'hydrogène, un atome d'halogène, des groupes hydroxy, alkyl en C₁ à C₆, alkoxy, en C₁ à C₆ et alcényle en C₁ à C₆, lesdits groupes alkyle en C₁ à C₆, alkoxy en C₁ à C₆ et alcényle en C₁ à C₆ pouvant être facultativement substitués avec un groupe choisi entre des groupes (alkoxy en C₁ à C₆) carbonyle, aryle et hétérocyclyle,
comprenant une ou plusieurs des étapes a), b), c) et d), dans lequel
l'étape a) comprend la résolution optique d'une énamine de formule 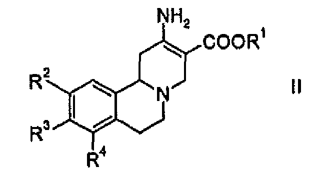 dans laquelle R², R³ et R⁴ répondent aux définitions précitées et R¹ représente un groupe alkyle en C₁ à C₆ ou benzyle, en présence d'un agent de résolution optique actif pour former le sel de (S)-énamine de formule 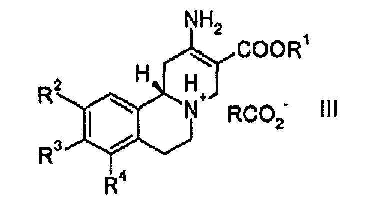 dans laquelle R¹, R², R³ et R⁴ répondent aux définitions précitées et RCO₂⁻ représente la base conjuguée de l'agent de résolution ;
l'étape b) comprend la transformation du sel de (S)-énamine de formule III en l'ester de formule 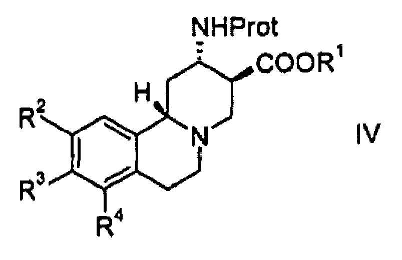 dans laquelle R¹, R², R³ et R⁴ répondent aux définitions précitées et Prot représente un groupe protecteur de la fonction amino ;
l'étape c) comprend l'amidation de l'ester de formule IV pour former l'amide de formule 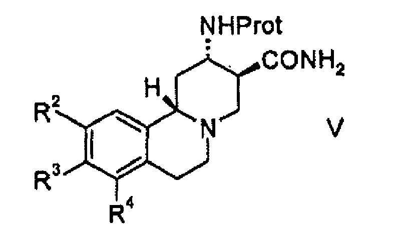 dans laquelle R², R³, R⁴ et Prot répondent aux définitions précitées ;
l'étape d) comprend la dégradation de l'amide de formule V pour former l'amine de formule 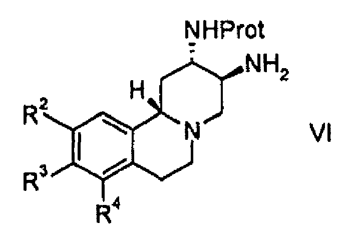 dans laquelle R², R³, R⁴ et Prot répondent aux définitions précitées ;
ledit procédé comprenant l'étape a) ; et
ledit procédé étant **caractérisé en ce que** la résolution optique dans l'étape a) est une résolution dynamique induite par cristallisation.

2. Procédé suivant la revendication 1, comprenant les étapes a) et b).

3. Procédé suivant la revendication 1, comprenant les étapes a) à d) .

4. Procédé suivant la revendication 1, dans lequel les étapes b) et c) sont mises en oeuvre sans isolement de l'intermédiaire IV.

5. Procédé suivant les revendications 1 à 4, **caractérisé en ce que** la résolution optique dans l'étape a) est effectuée avec un agent de résolution de formule 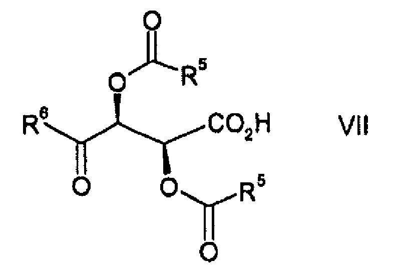 dans laquelle
R⁵ est choisi dans le groupe consistant en un groupe phényle non substitué, un groupe phényle substitué avec un, deux ou trois groupes choisis indépendamment entre des groupes alkyle en C₁ à C₆, alkoxy en C₁ à C₆ et halogéno, un groupe alkyle en C₁ à C₆, un groupe benzyle, dans lequel le noyau phényle est non substitué ou substitué avec un, deux ou trois groupes choisis indépendamment entre des groupes alkyle en C₁ à C₆, alkoxy en C₁ à C₆ et halogéno, et un groupe -NH-phényle, dans lequel le noyau phényle est non substitué ou substitué avec un, deux ou trois groupes choisis indépendamment entre des groupes alkyle en C₁ à C₆, alkoxy en C₁ à C₆ et halogéno ; et
R⁶ est choisi dans le groupe consistant en des groupes hydroxy, alkoxy en C₁ à C₆ et -NR⁷R⁸, dans lequel R⁷ et R⁸, indépendamment l'un de l'autre, représentent un groupe alkyle en C₁ à C₆.

6. Procédé suivant les revendications 1 à 5, **caractérisé en ce que** la résolution optique dans l'étape a) est effectuée avec un agent de résolution de formule 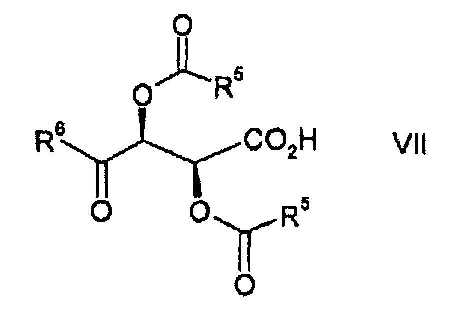 dans laquelle R⁵ est choisi dans le groupe consistant en un groupe phényle non substitué, un groupe phényle substitué avec un, deux ou trois groupes choisis indépendamment entre des groupes alkyle en C₁ à C₆, alkoxy en C₁ à C₆ et halogéno, et un groupe -NH-phényle, dans lequel le noyau phényle est non substitué ou substitué avec un, deux ou trois groupes choisis indépendamment entre des groupes alkyle en C₁ à C₆, alkoxy en C₁ à C₆ et halogéno, et
R⁶ représente un groupe hydroxy ou -NR⁷R⁸, dans lequel R⁷ et R⁸, indépendamment l'un de l'autre, représentent un groupe alkyle en C₁ à C₆.

7. Procédé suivant les revendications 1 à 6, **caractérisé en ce que** à résolution optique dans l'étape a) est effectuée avec un agent de résolution choisi entre l'acide (+)-o,o'-dibenzoyl-D-tartrique et le monodiméthylamide d'acide (+)-o,o'-dibenzoyl-D-tartrique.

8. Procédé suivant les revendications 1 à 7, **caractérisé en ce que** la résolution optique dans l'étape a) est effectuée dans un solvant choisi dans le groupe consistant en l'eau, le méthanol, l'éthanol, l'isopropanol, l'acétone, le tétrahydrofurane, l'acétate d'éthyle, le toluène et leurs mélanges.

9. Procédé suivant les revendications 1 à 8, **caractérisé en ce que** la résolution optique dans l'étape a) est effectuée avec l'énamine de formule II dans laquelle R¹ représente un groupe méthyle, éthyle ou isopropyle.

10. Procédé suivant la revendication 1, **caractérisé en ce que** la transformation du sel de (*S*)-énamine de formule III dans l'étape b) est effectuée par une réduction dans des conditions acide puis par introduction d'un groupe protecteur de la fonction amino.

11. Procédé suivant la revendication 10, **caractérisé en ce que** la réduction est effectuée avec des agents réducteurs choisis entre le borohydrure de sodium, le borohydrure de lithium et le cyanoborohydrure de sodium.

12. Procédé suivant la revendication 1, 10 ou 11, **caractérisé en ce que** la réduction est effectuée dans un solvant organique à des températures de -40°C à 30°C,

13. Procédé suivant la revendication 1, 10, 11 ou 12, **caractérisé en ce qu'**un groupe protecteur dans la fonction amino choisi dans le groupe consistant en les groupes trichloroéthoxycarbonyle, benzyloxycarbonyle, chloroacétyle, trifluoroacétyle, phénylacétyle, formule, acétyle, benzoyle, tertio-butoxycarbonyle, para-méthoxybenzyloxy-carbonyle, diphénylméthoxycarbonyle, phtaloyle, succinyle, benzyle, diphénylméthyle, triphénylméthyle, méthanesulfonyle, para-toluènesulfonyle, pivaloyle, triméthylsilyle, triéthylsilyle et triphénylsilyle est introduit.

14. Procédé suivant la revendication 1, **caractérisé en ce que** l'amidation dans l'étape c) est effectuée avec du formamide/méthylate de sodium, du formamide/éthylate de sodium, de l'acétamide/méthylate de sodium ou de l'acétamide/éthylate de sodium.

15. Procédé suivant la revendication 1 ou 14, **caractérisé en ce que** l'amidation dans l'étape c) est effectuée dans un solvant organique à des températures comprises dans l'intervalle de 10°C à 70°C.

16. Procédé suivant la revendication 1, **caractérisé en ce que** la dégradation de l'amide de formule V dans l'étape d) est effectuée suivant le principe de la dégradation d'Hofmann.

17. Procédé suivant la revendication 1 ou 16, **caractérisé en ce que** la dégradation de l'amide de formule V dans l'étape d) est effectuée avec un agent oxydant choisi entre le diacétate d'iodosobenzène, le bistrifluoro-acétate d'iodosobenzène et le bistrichloroacétate d'iodosobenzène.

18. Procédé suivant les revendications 1 à 17 pour la préparation de l'ester tertio-butylique d'acide (2*S*, 3*S*, 11b*S*)-(3-amino-9,10-diméthoxy-1,3,4,6,7,11b-hexahydro-2H-pyrido-[2,1-a]isoquinoline-2-yl)]-carbamique.

19. Procédé suivant les revendications 1 à 17, pour la préparation de la (*S*)-1-((2*S*, 3*S*, 11b*S*)-2-amino-9,10-diméthoxy-1,3,4,6,7,11b-hexahydro-2H-pyrido[2,1-a]isoquinoline-3-yl)-4-fluorométhyl-pyrrolidine-2-one.

20. Procédé suivant les revendications 1 à 17, pour la préparation de la (*S*)-1-((2*S*, 3*S*, 11b*S*)-2-amino-9,10-diméthoxy-1,3,4,6,7,11b-hexahydro-2H-pyrido[2,1-a]isoquinoline-3-yl)-4-fluorométhyl-pyrrolidine-2-one, comprenant le procédé suivant les revendications 1 à 17, suivi par
e) le couplage de l'ester tertio-butylique d'acide (2*S*, 3*S*, 11b*S*)-3-amino-9,10-diméthoxy-1,3,4,6,7,11b-hexahydro-2H-pyrido[2,1-a]isoquinoline-2-yl)-carbamique avec la (*S*)-4-fluorométhyl-dihydrofurane-2-one de formule 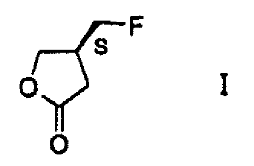
f) la cyclisation de l'ester tertio-butylique d'acide (2*S*, 3*S*, 11b*S*)-3-(3-fluorométhyl-4-hydroxy-butyrylamino)-9,10-diméthoxy-1,3,4,6,7,11b-hexahydro-2H-pyrido[2,1-]isoquinoline-2-yl]-carbamique obtenu en présence d'une base, et
g) la suppression de la protection de l'ester tertio-butylique d'acide (2*S*, 3*S*, 11b*S*)-3-((4*S*)-fluorométhyl-2-oxo-pyrrolidine-1-yl)-9,10-diméthoxy-1,3,4,6,7,11b-hexahydro-2H-pyrido[2,1-a]isoquinoline-2-yl]-carbamique obtenu.

21. Sel de (S)-énamine de formule 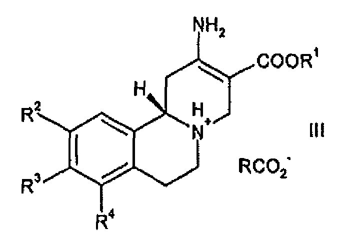 dans laquelle R¹, R², R³ et R⁴ répondent aux définitions dans la revendication 1 et RCO₂⁻ représente la base conjuguée de l'agent de résolution.

22. Ester de formule 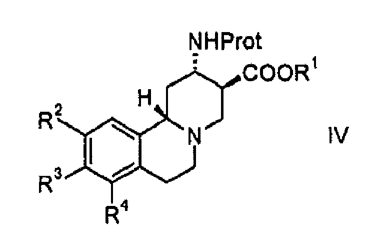 dans laquelle R¹, R², R³ et R⁴ répondent aux définitions dans la revendication 1 et Prot représente un groupe protecteur de la fonction amino,
